(19) Europäisches Patentamt
European Patent Office
Office européen des brevets

(11) EP 1 659 185 A1

(12) # EUROPÄISCHE PATENTANMELDUNG

(43) Veröffentlichungstag:
**24.05.2006 Patentblatt 2006/21**

(51) Int Cl.:
***C12Q 1/68*** *(2006.01)*

(21) Anmeldenummer: **04105949.4**

(22) Anmeldetag: **19.11.2004**

(84) Benannte Vertragsstaaten:
**AT BE BG CH CY CZ DE DK EE ES FI FR GB GR HU IE IS IT LI LU MC NL PL PT RO SE SI SK TR**
Benannte Erstreckungsstaaten:
**AL HR LT LV MK YU**

(71) Anmelder: **Stiftung Tierärztliche Hochschule Hannover**
**30559 Hannover (DE)**

(72) Erfinder:
- **Distl, Ottmar, Prof. Dr.**
  **30559 Hannover (DE)**
- **Rak, Gerlinde**
  **30173 Hannover (DE)**
- **Drögemüller, Cord, Dr.**
  **30169 Hannover (DE)**

(74) Vertreter: **Taruttis, Stefan Georg et al**
**Vahrenwalder Strasse 7**
**30165 Hannover (DE)**

(54) **Genetischer Test zur Taubheit von Hunden**

(57) Die vorliegende Erfindung betrifft ein Verfahren zur Bestimmung des Haplotyps eines Hundes in Bezug auf die congenitale sensorineurale Taubheit, gekennzeichnet durch die Kopplungsanalyse der Ausprägungen bestimmter Mikrosatelliten mit dem Phänotyp der sensorineuralen Taubheit.

EP 1 659 185 A1

## Beschreibung

Die vorliegende Erfindung bezieht sich auf einen Test von Hunden, mit dem festgestellt werden kann, ob ein Tier mit normalem Höhrvermögen ein rezessives Allel für die sensorineurale Taubheit trägt.

Der erfindungsgemäße Test verwendet Mikrosatelliten als genetische Marker für Gene, deren defekte Allele rezessiv zur sensorineuralen Taubheit führen.

Es ist bekannt, dass die congenitale sensorineurale Taubheit bei mehr als 54 verschiedenen Hunderassen auftritt. Insbesondere bei Dalmatinern tritt die vererbte Taubheit mit großer Häufigkeit auf, nämlich zwischen ca. 16 und 30% der Dalmatiner-Population zeigen einen einseitigen oder beidseitigen Höhrverlust. Es ist bekannt, dass die congenitale sensorineurale Taubheit bei Dalmatinern vererbt wird. Ein für die Taubheit verantwortliches Gen ist bisher bei Hunden nicht identifiziert worden. Es wird vermutet, dass ein rezessives Hauptgen neben der Wirkung polygener Komponenten und von Pigmentierungsgenorten Ursache für die Segregation dieser Taubheit ist (Juraschko et al., *Mammalian Genome* 14, 547-554 (2003)). So wurde gezeigt, dass ein Zusammenhang mit blauer Augenfarbe und dem Auftreten von Platten besteht, wie auch die von anderen Säugetieren bekannte Assoziation einer fleckigen oder vollständigen Depigmentierung der Haut und Haare sowie der Iris mit der sensorineuralen Taubheit und cochlearer Degeneration des Innenohrs besteht.

Weiterhin ist bekannt, dass der Vererbungsgang des für die congenitale sensorineurale Taubheit verantwortlichen Gens bzw. Gene rezessiv ist. In der Folge können auch phänotypisch gesunde Tiere, d.h. mit normalem Höhrvermögen ausgestattete, neben gesunden Allelen der verantwortlichen Gene deren defekte Allele tragen. Es ist daher für einen Züchter von großer Bedeutung, eine Vorhersage darüber treffen zu können, ob ein phänotypisch gesundes Tier Träger von defekten Allelen ist, die bei Nachkommen zur phänotypischen Taubheit führen, wenn sie homozygot für mindestens eines oder mehrere Defektallele sind.

Bisher ist kein molekulargenetischer Test bekannt, der eine Aussage darüber ermöglicht, ob ein Hund Träger von defekten Allelen ist, die zur congenitalen sensorineuralen Taubheit führen. Daher ist es die Aufgabe der vorliegenden Erfindung, einen molekulargenetischen Test zur Verfügung zu stellen, mit dem eine verlässliche Aussage darüber getroffen werden kann, ob ein Tier mit normalem Höhrvermögen dennoch Träger eines oder mehrerer defekter Gene ist, die bei Nachkommen zur congenitalen sensorineuralen Taubheit führen.

## Allgemeine Beschreibung der Erfindung

Die vorliegende Erfindung stellt einen genetischen Test bereit, mit dem eine Voraussage hoher Wahrscheinlichkeit darüber getroffen werden kann, ob ein phänotypisch gesundes Tier Träger von Allelen ist, die für die congenitale sensorineurale Taubheit (CST) verantwortlich sind. Voraussetzung dafür ist, dass zumindest ein Teil der Vorfahren oder Nachkommen dieses Hundes genetisch analysiert wurde und deren Phänotyp hinsichtlich des Höhrvermögens bekannt ist.

Die congenitale sensorineurale Taubheit ist tierschutzrelevant, dass heißt, Tiere, die einen identifizierbaren genetischen Defekt aufweisen oder bei deren Paarung Nachkommen mit diesem Defekt zu erwarten sind, sind von der weiteren Zucht auszuschließen.

Erfindungsgemäß werden hochpolymorphe Mikrosatelliten als Marker für das Gen bzw. die Gene bereitgestellt, deren defekte Allele für das Auftreten der congenitalen sensorineuralen Taubheit verantwortlich sind. Diese Mikrosatelliten sind eng mit dem für CST verantwortlichen Genort gekoppelt, möglicherweise intragenisch. Das Testprinzip liegt darin, den Haplotyp von Hunden eines Stammbaums mit Hilfe der erfindungsgemäßen Marker, d.h. der Mikrosatelliten festzustellen, sodass im Zusammenhang mit dem Phänotyp der Tiere eines Stammbaums die bestimmten Ausprägungen der identifizierten Marker zumindest einem Gendefekt für die congenitale sensorineurale Taubheit zugeordnet werden kann.

Im Rahmen dieser Erfindung bezieht sich der Ausdruck Hund oder Tier sowohl auf männliche als auch weibliche Individuen.

## Genaue Beschreibung der Erfindung

Die Erfinder konnten zeigen, dass bestimmte Mikrosatelliten eng mit Genen oder Genabschnitten gekoppelt sind, deren fehlerhafte Allele Ursache für die sensorineurale Taubheit bei Hunden sind. Aufgrund der engen Kopplung der erfindungsgemäßen Mikrosatelliten mit dem für CSD ursächlichen Genort oder Genorten wird vermutet, dass zumindest einige der Mikrosatelliten intragenisch in diesem Genort lokalisiert sind. So zeigen Vergleiche der Gensequenzen, die in der unmittelbaren Umgebung der erfindungsgemäßen Mikrosatelliten liegen, dass zumindest einige der Mikrosatelliten intragenisch in dem betreffenden Gen und oder in einer Distanz von weniger als 150 kb lokalisiert sind. Dies würde auch die Beobachtung erklären, dass einige der Mikrosatelliten im wesentlich nicht rekombinieren.

Erfmdungsgemäß wird die Kopplung der Ausprägungen, d.h. die Allele der spezifischen, erfindungsgemäß zu analysierenden Mikrosatelliten mit dem Phänotyp der Individuen analysiert, die zu einem Stammbaum gehören. Diese Zuordnung mittels einer Kopplungsanalyse von miteinander verwandten Tieren bekannten Phänotyps führt im Ergebnis zu einer Identifikation von solchen Mikrosatelliten, die defekte Allele der sensorineuralen Taubheit anzeigen. Die Analyse der Mikrosatelliten innerhalb eines Stammbaums ist deshalb erforderlich, um eine verläßliche Aussage darüber treffen zu können, welche Ausprägungen der spezifischen Mikrosatelliten für einen Gendefekt spezifisch sind, von dem nur der Phänotyp bekannt ist. Denn die Mikrosatelliten sind nur physikalisch mit dem Genort gekoppelt, von dem die sensorineurale Taubheit verursacht wird, so dass die Ausprägung der Mikrosatelliten erst nach Zuordnung zu einem Haplotyp als Marker für gesunde oder defekte Allele der Gene dienen kann, die zur sensorineuralen Taubheit führen. Diese erfindungsgemäßen Mikrosatelliten sind hochpolymorph, so dass sie für die interessierenden Allele der sensorineuralen Taubheit jeweils ein spezifisches Ausprägungsmuster, d.h. eine spezifische Größe (Allel) entsprechend der Anzahl der jeweiligen Struktureinheiten zeigen.

Die erfindungsgemäßen Mikrosatelliten, die bevorzugt als Marker verwendet werden, sind mit dem Gen für Claudin 14 (CLDN14), GJB2, GJB6, GJA1, EYA4, POU4F3, MYH9 bzw. dem Gen für Collagen 11A2 (COLL11A2) assoziiert. Die Kopplung ist signifikant mit einer Irrtumswahrscheinlichkeit von $p<0,02$ für COLL11A2 und $p<0,04$ für CLDN14. Eine Haplotypenanalyse ergab, dass bei allen untersuchten Dalmatinerfamilien entweder einer oder sogar beide der signifikant mit den erfindungsgemäßen Mikrosatelliten gekoppelten Genorte an der Entwicklung der sensorineuralen Taubheit beteiligt sind. Für die Identifizierung des Genotyps von Individuen eines Stammbaums ist daher die Zuordnung spezifischer Mikrosatelliten zu den Allelen der Gene, die für die sensorineurale Taubheit verantwortlich sind, ausreichend, wenn nur die mit Claudin 14 in Beziehung stehenden Mikrosatelliten oder die mit Collagen 11A2 in Beziehung stehenden Mikrosatelliten analysiert werden. Es ist jedoch bevorzugt, die Genauigkeit des Tests dadurch zu erhöhen, dass zumindest zwei der erfindungsgemäßen Mikrosatelliten auf ihre Kopplung mit dem Phänotyp der Taubheit analysiert werden.

Die Kopplung kann mit statistischen Verfahren analysiert werden, vorliegend wurde das Verfahren von Abecasis et al. *Nature Genetics* 30, 97-101 (2002) in Form des Rechenprogramms MERLIN, Version 0.10.2 (Center for Statistical Genetics, University of Michigan, MI, USA) für die Berechnung von Zmean und dessen Irrtumswahrscheinlichkeit verwendet. Der von Kong und Cox *(American Journal of Human Genetics* 61, 1179-1188 (1997)) aus Zmean abgeleitete Lod-Score und dessen Irrtumswahrscheinlichkeit wurden gleichzeitig ermittelt. Diese statistische Analyse der Kopplungsbeziehungen zwischen dem phänotypischen Merkmal der congenitalen sensorineuralen Taubheit und genomweit verteilten, hochpolymorphen Mikrosatelliten ist ein nichtparametrisches Verfahren. Dieses benötigt im Gegensatz zu den parametrischen Verfahren der Kopplungsanalyse keine Annahmen hinsichtlich der Art des Erbgangs und der Allelfrequenz in der untersuchten Population. Das nichtparametrische Verfahren beruht auf der Schätzung des Anteils der gemeinsamen Allele, welche die betroffenen Individuen innerhalb der jeweiligen Familien gemeinsam haben. Anschließend wird der Erwartungswert für den Anteil abstammungsidentischer Allele für jedes betroffene Familienmitglied berechnet und paarweise mit allen anderen betroffenen Familienmitgliedern verglichen. Die Abweichungen werden über alle paarweisen Vergleiche aufsummiert und in eine normalverteilte Variable mit Mittelwert Null und Standardabweichung 1 transformiert (Zmean). Dieser Wert stellt die Teststatistik dar, mit deren Hilfe eine signifikante Abweichung der Allelverteilung von dem erwarteten Anteil bei den gegebenen Verwandtschaftsbeziehungen zwischen den verglichenen verwandten Paaren mit congenitaler sensorineuraler Taubheit und zufälliger Aufteilung der Allele festgestellt werden kann. Bei der Berechnung der Teststatistik werden alle Marker eines Chromosoms gleichzeitig miteinbezogen, wodurch die Anordnung der Allele auf dem jeweiligen Chromosomenstrang berücksichtigt wird und somit die Wahrscheinlichkeit steigt, eine signifikante Kopplung zu entdecken. Dementsprechend bezieht sich die Irrtumswahrscheinlichkeit auf alle Marker eines Chromosoms.

Für die erfindungsgemäßen Mikrosatelliten wurden die Werte für PIC (polymorphism information content) als Maß für die Relevanz der Polymorphie errechnet als

$$PIC = 1 - \sum_{i=1}^{n} p_i^{\,2} - \sum_{i=1}^{n-1} \sum_{j=i+1}^{n} 2 p_i^{\,2} p_j^{\,2}$$

mit $i \neq j$ oder i = j und

$p_i$ = Allelfrequenz für das i-te Allel des betreffenden Mikrosatellitenmarkers

$p_j$ = Allelfrequenz für das j-te Allel des betreffenden Mikrosatellitenmarkers

$\Sigma$ = Summation über die n bzw. n-1 Allele des betreffenden Mikrosatellitenmarkers.

Der Vorteil erfindungsgemäßer Mikrosatelliten, die in den Tabellen 1 und 2 definiert sind, liegt darin, dass sie eng mit dem verantwortlichen Genbereich bzw. Genort gekoppelt sind, sodass sie im Vererbungsgang an die verantwortlichen Gene bzw. defekten Allele gekoppelt bleiben und andererseits, dass sie hochgradig polymorph sind. Polymorphie bedeutet, dass die mit den für das Höhrvermögen bzw. den defekten Allelen für die sensorineurale Taubheit verantwortlichen Genorten gekoppelten Mikrosatelliten dieselben definierten Struktureinheiten aufweisen und mit denselben Primerpaaren amplifiziert werden können, die Anzahl an Struktureinheiten jedoch von Stammbaum zu Stammbaum abweicht. Auf diese Weise ist für einen Stammbaum eine individuelle Zuordnung der Ausprägung dieser Mikrosatelliten möglich, d.h. der Länge ihrer spezifischen Amplifikate zu bestimmten Allelen, deren Defekt über die Stammbaumanalyse den bekannten Phänotypen zugeordnet werden kann.

Die CST ist genetisch heterogen, d.h. eines oder mehrere Gene können ursächlich sein. Die ursächlichen Defektallele können unter Hundefamilien variieren, d.h. unterschiedliche Gene betreffen. Daher ist für verwandte Tiere, vorzugsweise einen Stammbaum von Hunden, zunächst festzustellen, welche der erfindungsgemäßen Mikrosatelliten mit dem Phänotyp CST gekoppelt sind, um den Test auf diesen bzw. diese Mikrosatelliten beschränken zu können.

Zur Analyse der jeweiligen Mikrosatelliten sind Primerpaare geeignet, die stromauf und stromab von den jeweiligen Mikrosatelliten liegen und für diese spezifisch sind. Die in den obigen Tabellen angeführten Primerpaare sind zur Identifikation der Mikrosatelliten mit den defmierten Struktureinheiten spezifisch, es ist jedoch möglich, andere Primerpaare zu identifizieren, mit denen ebenfalls die erfindungsgemäßen Mikrosatelliten identifiziert und per Polymerase-Kettenreaktion (PCR) spezifisch amplifiziert werden können.

Die vorliegende Erfindung verwendet vorzugsweise die Polymerase-Kettenreaktion als Nachweisverfahren für die erfindungsgemäßen Mikrosatelliten. Bei der Ausführung der Erfindung reicht es generell aus, mit Primerpaaren, die für jeweils einen Mikrosatelliten spezifisch sind, diesen Bereich zu amplifizieren und mittels der Gel-Elektrophorese die Größe des Amplifikats zu bestimmen. Die Größe des Amplifikats wird durch die Anzahl der jeweils vorhandenen Strukturelemente bestimmt, einschließlich der stromauf und stromab flankierenden Bereiche mit den stromauf bzw. stromab verwendeten Primern. Die in den Tabellen 1 und 2 angegebenen Längen der Amplifikate der PCR enthalten daher neben der für einen Stammbaum spezifischen Anzahl der definierten Struktureinheiten die flankierenden Bereiche des Mikrosatelliten, die einschließlich der Primer-Sequenzen jeweils mit amplifiziert werden.

Die zur Identifikation von spezifischen Mikrosatelliten (MS) eines Satzes verwendeten Ziffern (MS_1, MS_2, MS_3) wurden willkürlich zugeordnet. Dabei bezieht sich MS_1 auf das jeweils kleinste gefundene Allel eines Mikrosatelliten und größere Zahlen auf die nächst größeren identifizierten. Diese Zuordnung erfolgte willkürlich und entspricht nicht
notwendigerweise der Anzahl der jeweiligen Struktureinheiten.

In Tabelle 2 werden auch Primer angegeben, mit denen diese bevorzugten Mikrosatelliten amplifizierbar sind. Dabei werden von den Seq. ID Nrn 1-140 jeweils eine gradzahlig bezeichnete Sequenz mit der nächst größeren ungradzahlig bezeichneten Sequenz paarweise kombiniert.

Tabelle 1: Fragmentlängen der Allele der Mikrosatelliten CLDN14 und COL11A2

| Mikrosatellit | Allel | Fragmentlänge in bp |
|---|---|---|
| CLDN14_MS1 | 1 | 106 |
| | 2 | 122 |
| | 3 | 126 |
| CLDN14_MS2 | 1 | 148 |
| | 2 | 150 |
| | 3 | 154 |
| CLDN14_MS3 | 1 | 124 |
| | 2 | 138 |
| | 3 | 144 |
| | 4 | 148 |
| COL11A2_MS1 | 1 | 140 |
| | 2 | 138 |
| | 3 | 148 |
| COL11A2_MS3 | 1 | 236 |
| | 2 | 242 |
| | 3 | 246 |

Tabelle 2: Erfindungsgemäße Mikrosatelliten

| Mikro-satellit (MS) | Herkunft des MS | Struktureinheit und Anzahl der Struktureinheiten | Primersequenzen (5'→3') | HT /°C | PCR-Produkt (bp) |
|---|---|---|---|---|---|
| CDH23 _MS1 | RPCI81-99C20 | Seq ID Nr. 141: $(TTTA)_{15}$ | Seq ID Nr 1: V: AATCAGGAGGGGTGAGTGTG Seq ID Nr 2: R: CCCCCAGCTCATACATTCTC | 62 | 175 |
| CDH23 _MS2_F2 | RPCI81-99C20 | Seq ID Nr. 142: $(ACC)_9(ATC)_3$ | Seq ID Nr 3: V: CCTGTACTGAATGCTTGAGG Seq ID Nr 4: R: CATCTCTAGAAGAAGCCTCC | 60 | 109 |

| | | | | | |
|---|---|---|---|---|---|
| CLDN14 _MS1 | RPCI81-97L17 | Seq ID Nr. 143: $(ATTT)_7(GTTT)_5$ | Seq ID Nr 5: V: TCACATAGCATTATATATGGAC Seq ID Nr 6: R: TTGAGATGGCTCTTACTGAG | 56 | 123 |
| CLDN14 _MS2 | RPCI81-97L17 | Seq ID Nr. 144: $(CA)_{21}$ | Seq ID Nr 7: V: TCGATGATGCTTTCTGGTTG Seq ID Nr 8: R: AGGCTGTGAAATGGATGGAG | 60 | 156 |
| CLDN14 _MS3 | RPCI81-97L17 | Seq ID Nr. 145: $(GA)_{20}$ | Seq ID Nr 9: V: GAGAAGCACCAGGCATAGG Seq ID Nr 10: R: TGGTTTAGCAAGGCTGTTCC | 60 | 146 |
| COCH _MS1 | RPCI81-321I4 | Seq ID Nr. 146: $(CTTT)_{\sim 20}$ | Seq ID Nr 11: V: TGACATACGGAGGACCAAGAG Seq ID Nr 12: R: CCCCTCCCTTGCTCTATCTC | 62 | 179 |
| COCH _MS2 | RPCI81-321I4 | Seq ID Nr. 147: $(GA)_{13}$ | Seq ID Nr 13: V: TGCCCCTCAGAGATAATCAC Seq ID Nr 14: R: CTTCAATTATACACACAGGTAC | 58 | 259 |
| COL11A2 _MS1 | RPCI81-24M6 | Seq ID Nr. 148: $(AC)_{20}(AG)_9$ | Seq ID Nr 15: V: TGAATATGGGGCTGAGGAAG Seq ID Nr 16: R: TTCTCCCTCTGCCTGTGTC | 60 | 151 |
| COL11A2 _MS3 | RPCI81-24M6 | Seq ID Nr. 149: $(CT)_{16}$ TT $(CT)_5$ | Seq ID Nr 17: V: GGTTTAGCACTGCCTTCAGC Seq ID Nr 18: R: CATTAAGCATCTGGCATGTGG | 62 | 241 |
| DFNA5 _MS1 | RPCI81-66C12 | Seq ID Nr. 150: $(AAAAT)_{\sim 25}$ | Seq ID Nr 19: V: GAAAACTCAGATTAGCCTGG Seq ID Nr 20: R: ATCTTGAGAGCAAAGGTTGTG | 58 | 219 |
| DFNA5 _MS2 | RPCI81-66C12 | Seq ID Nr. 151: $(CT)_{11}(GT)_4(CT)_2$ | Seq ID Nr 21: V: TGGTTAGGGCATGATTCCAG Seq ID Nr 22: R: CATGTATAAAGAGTAATGCCAG | 58 | 186 |
| DIAPH1 _MS1 | RPCI81-362I5 | Seq ID Nr. 152: $(ATTT(T))_{23}$ | Seq ID Nr 23: V: CGGGAGAGGGTTTGACTAC Seq ID Nr 24: R: CTCCGTATTGCTCATCTTTCC | 60 | 214 |
| DIAPH1 _MS2 | RPCI81-362I5 | Seq ID Nr. 153: $(AC)_{22}$ | Seq ID Nr 25: V: AGCTTCCCTTCTCTGAGAC Seq ID Nr 26: R: GAGAATAGAGTTTGTGCTCAG | 58 | 191 |
| EDN3 _MS1 | RPCI81-366E14 | Seq ID Nr. 154: $(TAGA)_{12}$ | Seq ID Nr 27: V: GCTAGGAAAAATCCGCAATG Seq ID Nr 28: R: GACCCCCTAGGACACCAAC | 60 | 147 |

Fortsetzung Tabelle 2:

| | | | | | |
|---|---|---|---|---|---|
| EDNRB _MS1 | Zemke et al.(1999) | Seq ID Nr. 155: $(GA)_{25}$ | Seq ID Nr 29: V: GAGAATTGGGCATGGGCAGA Seq ID Nr 30: R: TGACTTTATCACTGGTCTTTG | 58 | 131 |
| EYA4 _MS1 | RPCI81-301N19 | Seq ID Nr. 156: $(GT)_{10}(AT)_{13}$ | Seq ID Nr 31: V: TTATGCAGCCCATGACAATC Seq ID Nr 32: R: CAAGGGAACTCAAAGGCTTG | 58 | 258 |
| EYA4 _MS2 | RPCI81-301N19 | Seq ID Nr. 157: $(AG)_{21}$ | Seq ID Nr 33: V: TGGACCAGGTCAGTTTGTG Seq ID Nr 34: R: TCTGCCTGTGTCTCTGCC | 58 | 227 |
| GJA1 _MS1 | RPCI81-370A16 | Seq ID Nr. 158: $(GT)_{16}$ | Seq ID Nr 35: V: ATGGCATGAAGAGGATACCG Seq ID Nr 36: R: AGGACAGGTGACGGCTCTAC | 60 | 134 |
| GJA1 _MS2 | RPCI81-370A16 | Seq ID Nr. 159: $(AG)_{12}$ | Seq ID Nr 37: V: GCTAGTACTCGATTGTGGTC Seq ID Nr 38: R: TCATGGGTTGTGAGATCCAG | 60 | 190 |
| GJB2 _MS1 | RPCI81-133O22 | Seq ID Nr. 160: $(CA)_{12}$ | Seq ID Nr 39: V: TTAATTTGCTCGTCTTCCTG Seq ID Nr 40: R: TGTAAGCTCCACGGATCACC | 58 | 148 |
| GJB2 _MS2 | RPCI81-133O22 | Seq ID Nr. 161: $(GA)_{8}$ | Seq ID Nr 41: V: CTCTCTTGGTCTCCCTCTGC Seq ID Nr 42: R: GGGAGTAGGGGTGGAGTAGG | 62 | 195 |
| GJB6 _MS2 | RPCI81-343C15 | Seq ID Nr. 162: $(CTTT)_{\sim 20}$ | Seq ID Nr 43: V: GGTGTTTCCTTTCCTTTTCT Seq ID Nr 44: R: GGTGTTCTCTCCCTTTCTCT | 58 | 218 |
| GJB2+6 _MS1 | FH2324 | Seq ID Nr. 163: $(GAAA)_{\sim 25}$ | Seq ID Nr 45: V: CTCTATGAAAGGTGATTGCC Seq ID Nr 46: R: CAGCCATACAAATGAGAATTG | 58 | 260 |
| MITF _MS2 | RPCI81-119P24 | Seq ID Nr. 164: $(GT)_{12}$ | Seq ID Nr 47: V: CTACAGTGAATCAGCACAGAC Seq ID Nr 48: R: CAGCCTTGACTGTTTCTTTGG | 62 | 181 |
| MITF _MS3 | REN100J13 | Seq ID Nr. 165: $(CA)_{15}$ | Seq ID Nr 49 V: TGATTGACTCTACTTTACACA Seq ID Nr 50: R: TATATTAGGCGGTTTTCTTCT | 56 | 164 |
| MYH9 _MS2 | RPCI81-374A12 | Seq ID Nr. 166: $(AG)_{11}$ | Seq ID Nr 51: V: ACCCAGGTGGCCTGATTG Seq ID Nr 52: R: GCACGCACGTTCTCTCTTTC | 58 | 96 |

Fortsetzung Tabelle 2:

| | | | | | |
|---|---|---|---|---|---|
| MYH9 _MS3 | FH2293 | Seq ID Nr. 167: $(GAAA)_{\sim 43}$ | Seq ID Nr 53: V: GAATGCCCTTCACCTTGAAA Seq ID Nr 54: R: GGAAAAGGAGAGATGATGCC | 58 | 227 |
| MYO6 _MS2 | RPCI81-156P14 | Seq ID Nr. 168: $(AC)_{13}$ | Seq ID Nr 55: V: TCTTCCTTGGAAAGGGAACTC Seq ID Nr 56: R: TGCCCTAACACTTGGAATGG | 62 | 94 |
| MYO7A _MS1 | RPCI81-193O2 | Seq ID Nr. 169: $(TC)_{13}$ | Seq ID Nr 57: V: TGGTTAAAACATTAAACTTATAG Seq ID Nr 58: R: TAGTATATAGAGATGCAATGG | 56 | 293 |
| MYO7A _MS2 | RPCI81-193O2 | Seq ID Nr. 170: $(AC)_{12}$ | Seq ID Nr 59: V: CATTGGGTGCTTTCCTGTTC Seq ID Nr 60: R: TGGAGCTGCAGGTATAGCC | 60 | 166 |
| MYO7A _MS3 | AHTH298 | Seq ID Nr. 171: $(GT)_{12}$ | Seq ID Nr 61: V: CCAGGCATTCGAGGGTG Seq ID Nr 62: R: CAGAACTTGAGGAACCATAG | 56 | 100 |
| MYO15A _MS1 | RPCI81-362O13 | Seq ID Nr. 172: $(AC)_{18}$ | Seq ID Nr 63: V: CCATGAACTTTGTGGAACTGC Seq ID Nr 64: R: AAAGGGTTGCTGTGGAGATG | 62 | 137 |
| MYO15A _MS2 | RPCI81-362O13 | Seq ID Nr. 173: $(GT)_{12}$ | Seq ID Nr 65: V: AGGCAGGTTCATCTGTGTCC Seq ID Nr 66: R: TCCCAGACCCAGCTACATTC | 62 | 174 |
| OTOF _MS1 | RPCI81-198L15 | Seq ID Nr. 174: $(TA)_3(TG)_9(TA)_2$ $(CA)_2$ C $(TAAA)_5$ | Seq ID Nr 67: V: CAGCCAACTGTATTCTCCTTG Seq ID Nr 68: R: ATCTTGAGCCCTGCATTAGG | 62 | 197 |
| PAX3 _MS1 | RPCI81-257H23 | Seq ID Nr. 175: $(AAT)_{18}$ | Seq ID Nr 69: V: GAAGCGAGGAGAGACAGTCC Seq ID Nr 70: R: AAGGAAGCCTCCTGACAACC | 60 | 164 |
| PAX3 _MS2 | RPCI81-257H23 | Seq ID Nr. 176: $(CT)_{13}$ | Seq ID Nr 71: V: CAGGGTCAGGCTCTATGCTC Seq ID Nr 72: R: TCCTATCATCCGGCTTTGAC | 60 | 201 |
| POU4F3 _MS4 | G2C02.466 | Seq ID Nr. 177: $(TG)_{13}$ | Seq ID Nr 73: V: TCTGGATTGTGGTCACAACC Seq ID Nr 74: R: ACTGGACACTTCTTTCAGACG | 58 | 160 |
| SLC26A4 _MS2 | RPCI81-47P17 | Seq ID Nr. 178: $(CT)_{17}$ | Seq ID Nr 75: V: AAAGTGGCTGGTTCGGAAG Seq ID Nr 76: R: AGCAGCAGCATACATTCCTC | 58 | 297 |

Fortsetzung Tabelle 2:

| | | | | | |
|---|---|---|---|---|---|
| SOX10 _MS2 | RPCI81-505H2 | Seq ID Nr. 179: $(TAAA)_{14}$ | Seq ID Nr 77: V: AAGTAGATCCTATTATCGTGG Seq ID Nr 78: R: AGTTTCAGTGTCTGTTAAATAG | 56 | 267 |
| TECTA _MS1 | RPCI81-59C2 | Seq ID Nr. 180: $(GT)_3$ CC $(GT)_{19}$ | Seq ID Nr 79: V: CCGGATTTCTGAGGAGGC Seq ID Nr 80: R: CATGCTCTTCACCAGAACC | 58 | 140 |
| TECTA _MS2 | RPCI81-59C2 | Seq ID Nr. 181: $(ATTT)_{12}$ | Seq ID Nr 81: V: TCAGCATGGATTTTGTAAAATC Seq ID Nr 82: R: GGACTGCGTGGACATCTG | 58 | 276 |
| TMPRSS 3_MS1 | RPCI81-125P17 | Seq ID Nr. 182: $(TC)_8(CA)_5(CG)_2$ $(CA)_9$ | Seq ID Nr 83: V: ACACGGTTCTCGCTGATGTG Seq ID Nr 84: R: TGAAGGGGATTGAACAGAGG | 62 | 228 |
| TMPRSS 3 _MS2 | AHTH246 | Seq ID Nr. 183: $(GT)_{16}$ | Seq ID Nr 85: V: TTCATTCCGAGGTTCTAACTG Seq ID Nr 86: R: CACCATCTCGTAGCCTTTATC | 60 | 260 |
| FH2834 | CFA18 | | Seq ID Nr 87: V: GCAAGCTTTAAAATACCTTTCC Seq ID Nr 88: R: GCCTGAACTGATTGATGACC | | 268 |
| REN54P1 1 | CFA18 | | Seq ID Nr 89: V: GGGGGAATTAACAAAGCCTGAG Seq ID Nr 90: R: TGCAAATTCTGAGCCCCACTG | | 231 |
| FH3944 | CFA18 | | Seq ID Nr 91: V: CTGTTGAGGAACAGAGAGAAGC Seq ID Nr 92: R: TGTGTTAGAATGTTGCTTCGAC | | 462 |
| REN93E0 7 | CFA20 | | Seq ID Nr 93: V: TGAGGGCTGCCACTGTAAATA Seq ID Nr 94: R: GGCCCCCTCACCACTCC | | 169 |
| FH2158 | CFA20 | | Seq ID Nr 95: V: ATGGCCACATCACCCTAGTC Seq ID Nr 96: R: CTCTCTCTGCATCTCTCATGAA | | 274 |
| C01.251 | CFA01 | | Seq ID Nr 97: V: TACCACTGTCATTTTTCCATGC Seq ID Nr 98: R: AAGAGGATACCGGTGGCAG | | 146 |
| C00901 | CFA01 | | Seq ID Nr 99: V: TAAAGGTCCATTGATAGAGGA Seq ID Nr 100: R: TGATCCCAGGAGTTCATTCTT | | 105 |

Fortsetzung Tabelle 2:

| | | | | | |
|---|---|---|---|---|---|
| FH2309 | CFA01 | | Seq ID Nr 101: V: GACTGAGTTCTTTCAGCACAGTG Seq ID Nr 102: R: GGCAGCCTTATTATTCATGGA | | 380 |
| REN51C16 | CFA30 | | Seq ID Nr 103: V: CAGTTCATCCTTCCCCCTCTC Seq ID Nr 104: R: GTGCTAGTCTGGCTGTGCTCA | | 258 |
| REN89K14 | CFA30 | | Seq ID Nr 105: V: TTTGGGGCAGGGAGAGTGG Seq ID Nr 106: R: CCCCTGCAGCAAATGTGGTT | | 234 |
| REN37A15 | CFA21 | | Seq ID Nr 107: V: AGCAAGGTCCACAAGAGCC Seq ID Nr 108: R: GTAGTTCCAGTCACATCCT | | 182 |
| FH3398 | CFA21 | | Seq ID Nr 109: V: CAAAGTTCAAACAGGAATGG Seq ID Nr 110: R: GTTCTGTGCTGAGCATTGG | | 221 |
| FH3210 | CFA02 | | Seq ID Nr 111: V: CAAATGCTTTAGATCAGACATACG Seq ID Nr 112: R: CTGAAAAATCTGTAGCGATGC | | 375 |
| FH2890 | CFA02 | | Seq ID Nr 113: V: CCAGATTAACCAGGATGAGG Seq ID Nr 114: R: AATGGCAAGGATGCTACTCC | | 198 |
| REN262G24 | CFA05 | | Seq ID Nr 115: V: GCCCACAGATAAGGGGTTTT Seq ID Nr 116: R: GGAAGGGACAGAAGTTTTGC | | 158 |
| FH3278 | CFA05 | | Seq ID Nr 117: V: CTGCTCTTTGTAACCCATGC Seq ID Nr 118: R: AATGCCTACCAGGTGAAGG | | 324 |
| FH2019 | CFA11 | | Seq ID Nr 119: V: ACCCCCCTGATTTGCTTTAC Seq ID Nr 120: R: AGTCCCGGAATCGAGTCC | | 198 |
| C11.873 | CFA11 | | Seq ID Nr 121: V: CTGGCAGATTACAGGTAGC Seq ID Nr 122: R: GTTCTCCAAAGCACTCAT | | 133 |
| FH2309 | CFA01 | | Seq ID Nr 123: V: GACTGAGTTCTTTCAGCACAGTG Seq ID Nr 124: R: GGCAGCCTTATTATTCATGGA | | 380 |

Fortsetzung Tabelle 2:

| | | | | | |
|---|---|---|---|---|---|
| FH2793 | CFA01 | | Seq ID Nr 125: V: CTATGTGCACGCTGAGAGAG Seq ID Nr 126: R: TACCCATAAAGTTGGGCTTG | | 208 |
| DTR13.6 | CFA13 | | Seq ID Nr 127: V: CTTGATGGCACCACTCTAAT Seq ID Nr 128: R: GTCCCTCAAAACACAAACAT | | 358 |
| FH2348 | CFA13 | | Seq ID Nr 129: V: GCATGCAAAGGTGTTAATTGG Seq ID Nr 130: R: ACACAAGGAAGCTTTGGGG | | 430 |
| C13.391 | CFA13 | | Seq ID Nr 131: V: ATTTGAAGTCCGTGGCTCC Seq ID Nr 132: R: GAAACAAAGAGGCAGCGATC | | 172 |
| FH3494 | CFA13 | | Seq ID Nr 133: V: AAGCCTATTTCTCCCTCTGC Seq ID Nr 134: R: CATTCTATTTGCTCCTGTGG | | 226 |
| REN120P21 | CFA13 | | Seq ID Nr 135: V: GGTTATTGTAAAGTCTGAGTGTTG Seq ID Nr 136: R: AATAGACTATGTAACTGTCTCTGGC | | 172 |
| FH2537 | CFA10 | | Seq ID Nr 137: V: AAAAAGTGTAGAGCTTTCTTCAAA Seq ID Nr 138: R: ATTGAGACCCAAGACTGTTAGTG | | 169 |
| FH4081 | CFA10 | | Seq ID Nr 139: V: TAAATTCAATAAGCCATGGAGG Seq ID Nr 140: R: GGTAACAACCAGAGTGTGTGTG | | 494 |

V: Vorwärtsprimer

R: Rückwärtsprimer

HT: Hybridisierungstemperatur. Falls keine Angabe gemacht wird, kann die für eine spezifische Hybridisierung erforderliche HT von einem Fachmann nach üblichen Berechnungen und/oder experimentell bestimmt werden.

Die in Tabelle 2 als Indexzahlen angegebenen Anzahlen der Struktureinheiten stellen jeweils eine konkrete Ausprägung der Mikrosatelliten dar, die für die hier definierten Primer die angegebene Länge des PCR-Produkts ergibt. Diese Anzahl der Struktureinheiten und daraus resultierend die Länge des PCR-Produkts ist für die erfindungsgemäßen Mikrosatelliten sehr variabel, entsprechend der hohen Polymorphie.

Die Bezeichnung der Mikrosatelliten erfolgte nach ihrer Kopplung mit einem Kandidatengen, das als sequenzhomolog identifiziert wurde. Diese sind in Tabelle 3 zusammengestellt, einschließlich Genen, für die selbst oder deren Homologe beim Hund ebenfalls Mikrosatelliten identifiziert werden können, die beim Hund mit CST gekoppelt sind.

Tabelle 3: Gene, deren Mikrosatelliten mit CST beim Hund gekoppelt sind

| Kurzbezeichnung | Gen |
|---|---|
| CDH23 | *Cadherin related 23* |
| CLDN14 | Claudin 14 |
| COCH | *Coagulation factor C homolog,* Cochlin |
| COL11A2 | Collagen, Typ XI, alpha 2 |
| DFNA5 | *Deafness,* autosomal dominant 5 |
| DIAPH1 | *Diaphanous homolog 1 (Drosophila)* |
| EDN3 | Endothelin 3 |
| EDNRB | Endothelin Rezeptor Typ B |
| EYA4 | *Eyes absent homolog 4 (Drosophila)* |
| GJA1 | *Gap junction protein*, alpha 1, 43kD (Connexin 43) |
| GJB2 | *Gap junction protein*, beta 2, 26kD (Connexin 26) |
| GJB6 | *Gap junction protein,* beta 6 (Connexin 30) |
| MITF | Microphthalmia assoziierter Transkriptionsfaktor |
| MYH9 | Myosin, *heavy polypeptide 9, non-muscle* |
| MY06 | Myosin VI |
| MYO7A | Myosin VIIA |
| MYO15A | Myosin XVA |
| OTOF | Otoferlin |
| PAX3 | *Paired box* Gen 3 (Waardenburg Syndrom 1) |
| POU4F3 | POU Domaine, Klasse 4, Transkriptionsfaktor 3 |
| SLC26A4 | *Solute carrier* Familie 26, Mitglied 4 |
| SOX10 | SRY *(sex determining region* Y)-*box 10* |
| TECTA | Tectorin alpha |
| TMPRSS3 | Transmembran Protease, Serin 3 |
| GJB3 | *Gap junction* Protein, beta 3, (Connexin 31) |
| PDS | Pendrin |
| PRES | Prestin |
| TMIE | *Transmembrane Inner Ear-Expressed Gene* |
| TMC1 | *Transmembrane Cochlear-Expressed Gene* 1 |
| STRC | Stereocilin |
| USH1C | USH1C Gen |
| MYO3A | Myosin IIIA |
| ESPN | Maus Homologes von Espin |
| WHRN | Whirlin |
| MYH14 | *Myosin, Heavy Chain 14, Nonmuscle* |
| WFS1 | *WFS1 Gen, Wolframin* |
| ACTG1 | Actin, Gamma-1 |

Tabelle fortgesetzt

| Kurzbezeichnung | Gen |
|---|---|
| TFCP2L3 | *Transcription Factor Cellular Promoter 2-Like 3* |
| MYO1A | Myosin IA |

Für eine Überprüfung der Identität der mittels der PCR identifizierten Amplifikate kann neben der Analyse der Fragmentlänge der Amplifikate zusätzlich die Identität ihrer Strukturelemente per Sequenzierung erfolgen und/oder eine Hybridisierung mit DNA-Sonden vorgenommen werden, die für die Strukturelemente jeweils spezifisch sind. Alternativ kann eine Hybridisierung mit den DNA-Sonden vorgenommen werden, die für die flankierenden Bereiche der Struktureinheiten spezifisch sind, die von den eingesetzten Primer-Paaren noch mit amplifiziert werden.

Der Nachweis solcher Sonden erfolgt mittels deren bekannter Markierung, beispielsweise radioaktiv, mit einem Fluoreszenz-Farbstoff (z.B. IRD) oder Antigen bzw. Enzym, mit einer anschließenden Nachweisreaktion. Die Zuordnung einer bestimmten Ausprägung eines Mikrosatelliten bzw. des Ausprägungsmusters der Mikrosatelliten, zu den Allelen, die mit diesen Mikrosatelliten gekoppelt sind und für die congenitale sensorineurale Taubheit verantwortlich sind, erfolgt erfmdungsgemäß durch eine Kopplungsanalyse von genetischem Material einer Hundefamilie. Dabei werden für einen Stammbaum, dessen Individuen für Taubheit segregieren, die erfindungsgemäßen Mikrosatelliten als Marker typisiert. Als Ergebnis der Kopplungsanalyse werden die Haplotypen der erfindungsgemäßen Mikrosatelliten in diesem Stammbaum identifiziert und auf gemeinsame Vererbung (Kosegregation) mit der phänotypisch festgestellten Taubheit überprüft. In der Konsequenz lässt sich dann bestimmen, ob ein Haplotyp der Ausprägung der Sätze von Mikrosatelliten mit gesunden oder defekten Allelen, die für die sensorineurale Taubheit codieren, gekoppelt ist.

Im nachfolgenden Beispiel wird an Hand von vier Dalmatiner-Familien gezeigt, dass sich mittels der erfindungsgemäßen Mikrosatelliten als Marker eine Aussage großer Wahrscheinlichkeit treffen läßt, ob ein phänotypisch gesundes Tier Träger eines defekten Allels ist. Dabei zeigen

- Figur 1 die Haplotypen von Dalmatiner-Familien von mit Claudin 14 gekoppelten Mikrosatelliten und
- Figur 2 die Haplotypen von Dalmatiner-Familien von mit Collagen 11A2 gekoppelten Mikrosatelliten.

Weibliche Tiere sind als als ● bzw. ○, männliche als ■ bzw. □ dargestellt, wobei schwarze Symbole ein- oder beidseitig taube Tiere und offene Symbole Tiere mit Hörvermögen bedeuten. Tiere mit unbekanntem Genotyp sind mit Diagonalstrich gekennzeichnet. Den Ausprägungen der Mikrosatelliten wurden willkürlich aufsteigende Ziffern für zunehmende Länge des Amplifikats zugeordnet.

<u>Beispiel: Kopplungsanalyse für die mit Claudin 14 und Collagen 11A2 assoziierten Mikrosatelliten zur phänotypischen CST bei Dalmatinerfamilien</u>

Das nachfolgende Beispiel erläutert im Detail, wie fünf für Taubheit segregierende Dalmatinerfamilien typisiert wurden, um die erfindungsgemäßen Mikrosatelliten einem gesunden oder defekten Genort der CST zuzuordnen. Die Gesamtzahl der Tiere betrug 55 mit Voll- und Halbgeschwistern, davon waren 24 von CST betroffen. Das Hörvermögen der Dalmatiner wurden von Tierärzten über die Messung audiometrisch evozierter Potentiale (AEP) festgestellt.

Genomische DNA wurde aus EDTA Blutproben mit dem QIAamp 96 Blood Kit (Qiagen, Hilden) isoliert.

Für die Identifikation von Mikrosatelliten wurden BAC-Klone, die eines Kandidatengene von Tabelle 3 enthalten, mit dem Qiagen Plasmid maxi kit (Qiagen, Hilden) isoliert, mit zwei von drei Enzymen (EcoRI, SacI, XbaI) restringiert und auf 0,8 % Agarosegelen elektrophoretisch getrennt. Die Fragmente wurden subkloniert (pGEM, Promega, Mannheim) und in *E. coli* (XL1-blue) transformiert. Plasmid-DNA wurde sequenziert und mit dem Sequencher Programm (Amersham, Freiburg) analysiert. Die Mikrosatelliten wurden manuell identifiziert. Primer konnten mit dem Programm Primer3 Input ausgewählt werden, vorzugsweise nach Identifikation repetitiver DNA Elemente, z.B. LINE (long interspersed nuclear element) oder SINE (short interspersed nuclear element).

Die Mikrosatelliten wurden mit den spezifischen Primern, die in Tabelle 2 angegeben sind, per Polymerase-Kettenreaktion unter den nachfolgend angegebenen Bedingungen amplifiziert und anschließend per Polyacrylamid-Gelelektrophorese hinsichtlich ihrer Amplifikatgröße analysiert. Als Ausgangsmaterial für die PCR wurde genomische DNA verwendet, die aus EDTA-Blutproben isoliert wurde, es können jedoch auch DNA-Proben aus Haar oder Gewebe bzw. Abstrichen verwendet werden.

PCR-Ansätze

| Bestandteil | μL |
|---|---|
| Wasser (ultrarein) | 7,26 |
| 10x Puffer (Q-Biogene) | 1,2 |
| DMSO, 99,5% | 0,24 |
| Vorwärtsprimer [10 pmol/μL] | 0,5 |
| Rückwärtsprimer [10 pmol/μL] | 0,5 |
| dNTPs [je 100 μM] | 0,2 |
| Taq-Polymerase [5 U/μL] | 0,1 |
| DNA [10 ng/μL] | 2 |

Auf Basis einer Wahrscheinlichkeitsrechnung mit nichtparametrischer Auswertung (Rechenprogramm MERLIN, Version 0.10.2) ist gefunden worden, dass in den analysierten Familien die folgenden Ausprägungen der analysierten Mikrosatelliten mit CST gekoppelt sind:

In Figur 1 zeigt die Familie 1 bei Analyse der Haplotypen von Muttertier 17 und Nachkommen 37 und 38 bzw. Tier 48, dass die Kombination des mütterlichen Allels 2 von CLDN14_MS1 mit Allel 3 von CLDN14_MS2 und Allel 3 CLDN14_MS3 mit CST gekoppelt ist. Ein weiteres Beispiel sind die Nachkommen 24 bis 26 mit den Eltern 11 (Vater) und 10 (Mutter). Hier sind die mütterlichen und väterlichen Allele 3 von CLDN14_MS2 und CLDN14_MS3 mit CST gekoppelt. Die übrigen Haplotypen sind weniger aussagekräftig, da diese homozygot sind und keines der Elterntiere analysiert werden konnte.

In Figur 2 wird deutlich, dass alle tauben Nachkommen des Muttertiers 10 denselben Haplotyp aufweisen, so dass hier Allel 2 von COL11A2_MS1 in Kombination mit Allel 2 von COL11A2_MS2 mit CST gekoppelt ist. Wenn überdies angenommen wird, dass der Haplotyp 2 (COL11A2_MS1), 2 (COL11A2_MS2) des nicht tauben Tiers 26 der nicht mit CST gekoppelte, jedoch gleich ausgeprägte Mikrosatellitensatz von Muttertier 10 ist, dann sind auch die übrigen Phänotypen dieser Gruppe plausibel.

Der Übertragungsweg vom Rüden 18 auf die tauben Nachkommen ist nicht durchgängig konsistent unter den genotypisierten Geschwistern, jedoch teilen alle tauben Tiere innerhalb eines Wurfs dieselben Ausprägungen dieser Mikrosatelliten.

Familie 3 war in Bezug auf COL11A2 nicht informativ, da kein Elterntier genotypisiert werden konnte und alle Nachkommen für den Mikrosatelliten COL11A2_MS3 homozygot sind. Daher ist es in diesem Fall nicht möglich, die korrekten Haplotypen allein auf Basis von COL11A2_MS1 zu berechnen.

Für den Rüden 14 in Familie 1 gibt es für CLDN14 und COL11A2 keine offensichtliche Beziehung mit CST für die halbgeschwisterlichen Nachkommen. In dieser Untergruppe haben einige der tauben und hörenden Tiere die gleichen Haplotypen von ihrem gemeinsamen Elter geerbt. Bei den Tieren 28, 31 und 34 könnte dies im falsch bestimmten Phänotyp oder in vertauschten Blutproben begründet sein. Jedoch würde dies im Fall von COL11A2 nicht erklären, weshalb die tauben Tiere nicht denselben Haplotyp des gemeinsamen Elters aufweisen. Eine weitere, nicht auszuschließende Möglichkeit ist die Rekombination, obwohl dies vorliegend durch die die Gene flankierenden Mikrosatelliten, d.h. die enge Kopplung der Mikrosatelliten mit den jeweiligen Genen, minimiert worden ist. Diese Beobachtung kann auch dadurch verursacht sein, dass CST vom Zusammenwirken mehrerer Gene bestimmt wird.

Daher wird bevorzugt, die Kopplung mehrerer Mikrosatelliten mit der CST zu analysieren und für die Analyse hörender Tiere auf die Eigenschaft zu testen, heterozygot Merkmalsträger zu sein.

Dieses Beispiel der Haplotypenanalyse zeigt, dass die Mikrosatelliten, die mit den Genen CLDN14 und COL11A2 gekoppelt sind, als Marker für ein Defektallel verwendet werden können, das homozygot zur CST führt. Weiterhin wird deutlich, dass auch mit beiden Genen, CLDN14 und COL11A2, in Kombination gekoppelte Mikrosatelliten das Vorliegen von Defektallelen, die zur CST führen, in bevorzugter Weise anzeigen können. Daher ist es bevorzugt, wenigstens zwei Sätze von Mikrosatelliten für die Analyse auf Kopplung mit einem Defektallel, das für CST verantwortlich ist, zu verwenden, die jeweils mit einem der Gene gekoppelt sind, die in Tabelle 3 aufgeführt sind.

## Patentansprüche

1. Mikrosatellit beim Hund oder Wolf, **dadurch gekennzeichnet, dass** dieser mit einem Defektallel gekoppelt ist, das zumindest als eines von mehreren Defektallelen für die congenitale sensorineurale Taubheit (CST) ursächlich ist, wobei der Mikrosatellit durch die nichtparametrische Kopplungsanalyse der Ausprägungen von Mikrosatelliten mit dem Phänotyp der CST identifizierbar ist.

2. Mikrosatellit nach Anspruch 1, der hochpolymorph und mit einem Gen gekoppelt ist, das aus der Gruppe ausgewählt ist, die aus CDH23, CLDN14, COCH, COL11A2, DFNA5, DIAPH1, EDN3, EDNRB, EYA4, GJA1, GJB2, GJB6, MITF, MYH9, MY06, MYO7A, MYO15A, OTOF, PAX3, POU4F3, SLC26A4, SOX10, TECTA, TMPRSS3, GJB3, PDS, PRES, TMIE, TMC1, STRC, USH1C, MYO3A, ESPN, WHRN, MYH14, WFS1, ACTG1, TFCP2L3 und MYO1A besteht.

3. Mikrosatellit nach Anspruch 1 oder 2, **gekennzeichnet durch** die Amplifizierbarkeit aus genomischer DNA von Hund oder Wolf **durch** die Primerpaare, die aus den Sequenzen Seq.-ID Nr. 1 bis Seq.-ID Nr. 140 und Homologen dieser ausgewählt sind.

4. Mikrosatellit nach einem der vorangehenden Ansprüche, **gekennzeichnet durch** die Verwendung zur Analyse der Kopplung mit einem Defektallel, das für die congenitale sensorineurale Taubheit kodiert.

5. Mikrosatellit nach einem der vorangehenden Ansprüche, **gekennzeichnet durch** eine Struktureinheit einer der Sequenzen Seq.-ID Nr. 141 bis Seq.-ID Nr. 183.

6. Einzelsträngige Nukleinsäure zur Verwendung als Primer, **gekennzeichnet durch** die Verwendung zur Analyse von Mikrosatelliten nach einem der vorangehenden Ansprüche.

7. Nukleinsäure nach Anspruch 6, **gekennzeichnet dadurch, dass** sie aus den Sequenzen der Seq ID Nr 1 bis Seq ID Nr. 140 und Homologen dieser ausgewählt sind.

8. Verfahren zur Bestimmung des Haplotyps eines Hundes oder Wolfs, **gekennzeichnet durch** die Verwendung zumindest eines Mikrosatelliten nach einem der Ansprüche 1 bis 5 oder einer Nukleinsäure nach einem der Ansprüche 6 oder 7.

9. Verfahren nach Anspruch 8, **gekennzeichnet dadurch, dass** der Haplotyp zumindest eines Mikrosatellits bei der Analyse von Hunden oder Wölfen eines Stammbaums auf die Kopplung mit dem Phänotyp der CST überprüft wird.

Figur 1

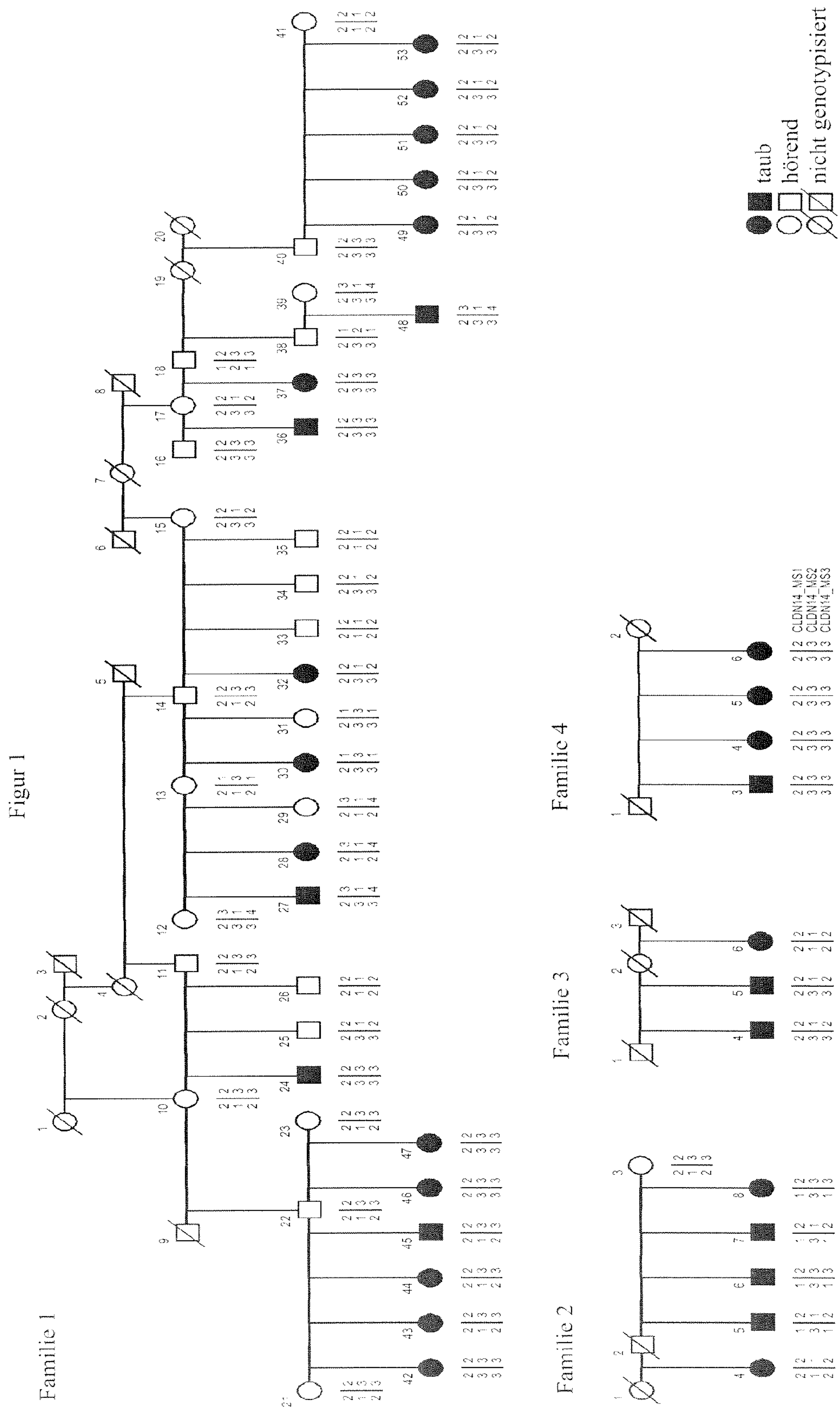
Familie 1
Familie 2
Familie 3
Familie 4
CLDN14_MS1
CLDN14_MS2
CLDN14_MS3
taub
hörend
nicht genotypisiert

Figur 2

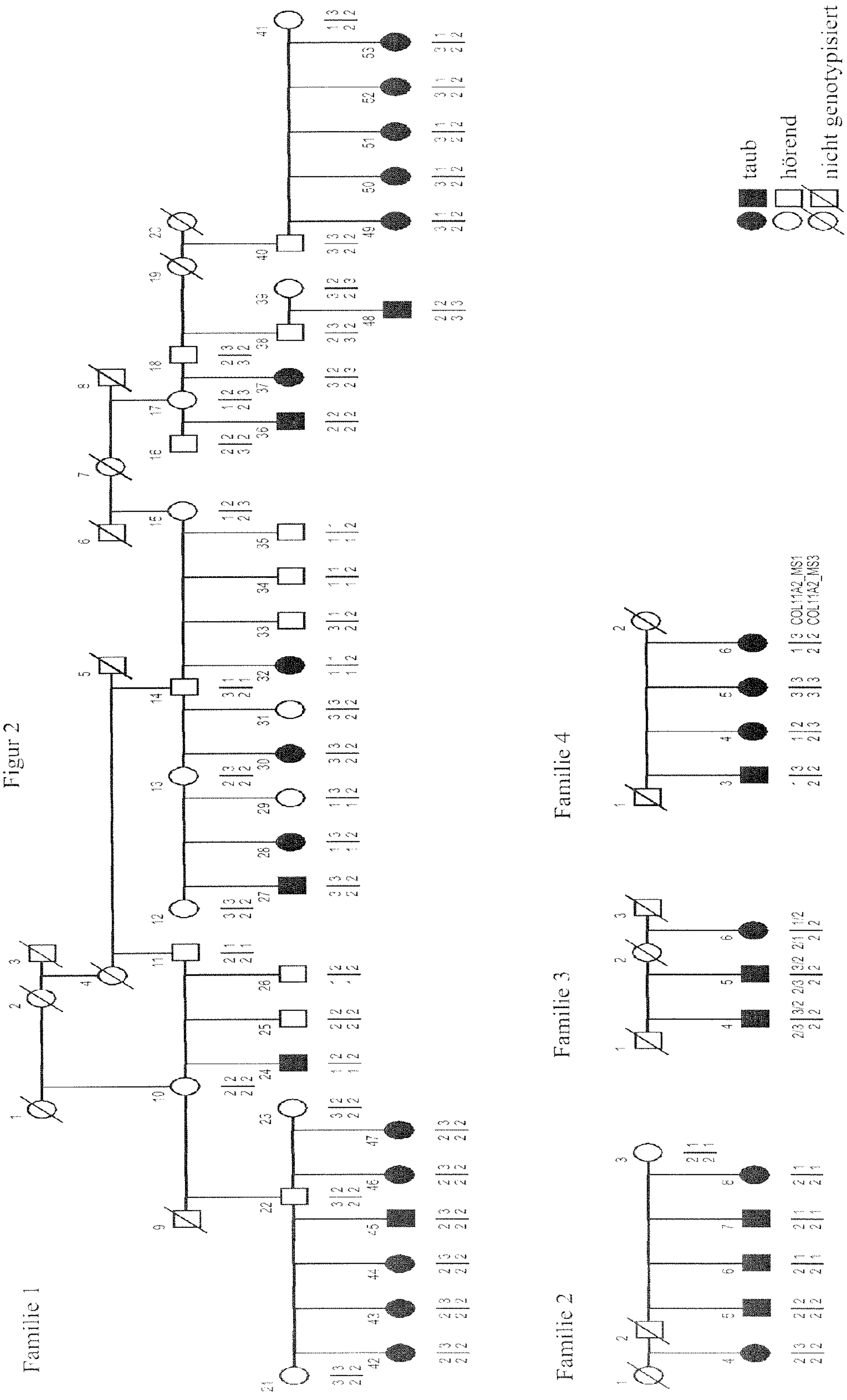
Familie 1
Familie 2
Familie 3
Familie 4
COL11A2_MS1
COL11A2_MS3
taub
hörend
nicht genotypisiert

Europäisches Patentamt

# EUROPÄISCHER RECHERCHENBERICHT

Nummer der Anmeldung
EP 04 10 5949

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | KUIPER H ET AL: "Assignment of the canine cadherin related 23 gene (CDH23) to chromosome 4q12-->q13 by fluorescence in situ hybridization and radiation hybrid mapping." CYTOGENETIC AND GENOME RESEARCH. 2002, Bd. 97, Nr. 1-2, 2002, Seite 140B, XP002324616 ISSN: 1424-8581 * das ganze Dokument * ----- | 1-9 | C12Q1/68 |
| X | RAK S G ET AL: "Chromosomal assignment of 20 candidate genes for canine congenital sensorineural deafness by FISH and RH mapping." CYTOGENETIC AND GENOME RESEARCH. 2003, Bd. 101, Nr. 2, 2003, Seiten 130-135, XP008045157 ISSN: 1424-859X * Seite 130 - Seite 131 * * Tabelle 2 * * Seite 134 * ----- | 1-9 | RECHERCHIERTE SACHGEBIETE (Int.Cl.7) |
| A | HOLMES N G: "MICROSATELLITE MARKERS AND THE ANALYSIS OF GENETIC DISEASE" BRITISH VETERINARY JOURNAL, BAILLIERE TINDALL, LONDON, GB, Bd. 150, Nr. 5, 1. September 1994 (1994-09-01), Seiten 411-421, XP000645761 ISSN: 0007-1935 * das ganze Dokument * ----- -/-- | | C12Q |

~~Der vorliegende Recherchenbericht wurde für alle Patentan~~sprüche erstellt

| Recherchenort | Abschlußdatum der Recherche | Prüfer |
|---|---|---|
| Den Haag | 19. April 2005 | Bellmann, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03) 3

Europäisches Patentamt

# EUROPÄISCHER RECHERCHENBERICHT

Nummer der Anmeldung
EP 04 10 5949

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| A | CARGILL E J ET AL: "Heritability and segregation analysis of deafness in U.S. Dalmatians." GENETICS. MAR 2004, Bd. 166, Nr. 3, März 2004 (2004-03), Seiten 1385-1393, XP002323605 ISSN: 0016-6731 * Seite 1385 * * Seite 1392 * ----- | | |
| A | CARGILL EDWARD J ET AL: "Multiplexing of canine microsatellite markers for whole-genome screens." GENOMICS. SEP 2002, Bd. 80, Nr. 3, September 2002 (2002-09), Seiten 250-253, XP002323625 ISSN: 0888-7543 * das ganze Dokument * ----- | | |
| A | HOLMES N G ET AL: "CHARACTERIZATION OF CANINE MICROSATELLITES" EXS, BIRKHAEUSER VERLAG, BASEL, CH, Bd. 67, 1993, Seiten 415-420, XP000645561 ISSN: 1023-294X * das ganze Dokument * ----- | | RECHERCHIERTE SACHGEBIETE (Int.Cl.7) |
| A | HOLMES N G ET AL: "EIGHTEEN CANINE MICROSATELLITES" ANIMAL GENETICS, BLACKWELL SCIENTIFIC PUBLICATIONS, LONDON, GB, Bd. 26, Nr. 2, 1. April 1995 (1995-04-01), Seiten 132-133, XP000645598 ISSN: 0268-9146 * das ganze Dokument * ----- | | |

~~Der vorliegende Recherchenbericht wurde für alle Patentans~~prüche erstellt

3

| Recherchenort | Abschlußdatum der Recherche | Prüfer |
|---|---|---|
| Den Haag | 19. April 2005 | Bellmann, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches
Patentamt

Nummer der Anmeldung
EP 04 10 5949

## GEBÜHRENPFLICHTIGE PATENTANSPRÜCHE

Die vorliegende europäische Patentanmeldung enthielt bei ihrer Einreichung mehr als zehn Patentansprüche.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

Erfindung 1: Ansprüche: 1-9 (teilweise)

Europäisches Patentamt

# MANGELNDE EINHEITLICHKEIT DER ERFINDUNG ERGÄNZUNGSBLATT B

Nummer der Anmeldung

EP 04 10 5949

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Erfindung 1: Ansprüche: 1-9 (teilweise)

Mikrosatellit, gekennzeichnet durch eine Struktureinheit der SEQ ID No: 141, der mit einem Primerpaar bestehend aus einem Primer mit SEQ ID No.1 und einem Primer mit SEQ ID No. 2 amplifizierbar ist, Primer mit SEQ ID No.1 und 2 und Verfahren zur Bestimmung des Haplotypes eines Hundes oder Wolfs unter Verwendung dieses Mikrosatelliten.

---

Erfindungen 2 bis 43: Ansprüche: 1-9 (teilweise)

Mikrosatellit, gekennzeichnet durch eine Struktureinheit der SEQ ID No: n, wobei n=142 bis 183, der mit einem Primerpaar bestehend aus einem Primer mit SEQ ID No.k, wobei k=3,5,7,9,11 bis 85 (k ist eine ungerade Zahl zwischen 3 und 85) und einem Primer mit SEQ ID No. k+1 amplifizierbar ist, Primer mit SEQ ID No.k und k+1 und Verfahren zur Bestimmung des Haplotypes eines Hundes oder Wolfs unter Verwendung dieses Mikrosatelliten.

---

Erfindungen 44 bis 70: Ansprüche: 1-9 (teilweise)

Mikrosatellit, der mit einem Primerpaar bestehend aus einem Primer mit SEQ ID No.k, wobei k=87,89,91 bis 139 (k ist eine ungerade Zahl zwischen 87 und 139) und einem Primer mit SEQ ID No. k+1 amplifizierbar ist, Primer mit SEQ ID No.k und k+1 und Verfahren zur Bestimmung des Haplotypes eines Hundes oder Wolfs unter Verwendung dieses Mikrosatelliten.

---

SEQUENCE LISTING

<110> Stiftung Tierärztliche Hochschule Hannover

<120> Genetischer Test zur Taubheit von Hunden

<130> N1005EP

<160> 183

<170> PatentIn version 3.1

<210> 1

<211> 20

<212> DNA

<213> artificial sequence

<220>

<223> Forward primer/vorwärts Primer

<400> 1
aatcaggagg ggtgagtgtg 20

<210> 2

<211> 20

<212> DNA

<213> artificial sequence

<220>

<223> Backward primer/rückwärts Primer

<400> 2
cccccagctc atacattctc 20

<210> 3

<211> 20

<212> DNA

<213> artificial sequence

<220>

<223> Forward primer/vorwärts Primer

<400> 3
cctgtactga atgcttgagg 20

<210> 4

<211> 20

<212> DNA

<213> artificial sequence

<220>

<223> Backward primer/rückwärts Primer

<400> 4
catctctaga agaagcctcc 20

<210> 5

<211> 22

<212> DNA

<213> artificial sequence

<220>

<223> Forward primer/vorwärts Primer

<400> 5
tcacatagca ttatatatgg ac 22

<210> 6

<211> 20

<212> DNA

<213> artificial sequence

<220>

<223> Backward primer/rückwärts Primer

<400> 6
ttgagatggc tcttactgag 20

<210> 7

<211> 20

<212> DNA

<213> artificial sequence

<220>

<223> Forward primer/vorwärts Primer

<400> 7
tcgatgatgc tttctggttg 20

<210> 8

<211> 20

<212> DNA

<213> artificial sequence

<220>

<223> Backward primer/rückwärts Primer

<400> 8
aggctgtgaa atggatggag 20

<210> 9

<211> 19

<212> DNA

<213> artificial sequence

<220>

<223> Forward primer/vorwärts Primer

<400> 9
gagaagcacc aggcatagg 19

<210> 10

<211> 20

<212> DNA

<213> artificial sequence

<220>

<223> Backward primer/rückwärts Primer

<400> 10
tggtttagca aggctgttcc 20

<210> 11

<211> 21

<212> DNA

<213> artificial sequence

<220>

<223> Forward primer/vorwärts Primer

<400> 11
tgacatacgg aggaccaaga g 21

<210> 12

<211> 20

<212> DNA

<213> artificial sequence

<220>

<223> Backward primer/rückwärts Primer

<400> 12
cccctcccctt gctctatctc 20

<210> 13

<211> 20

<212> DNA

<213> artificial sequence

<220>

<223> Forward primer/vorwärts Primer

<400> 13
tgcccctcag agataatcac 20

<210> 14

<211> 22

<212> DNA

<213> artificial sequence

<220>

<223> Backward primer/rückwärts Primer

<400> 14
cttcaattat acacacaggt ac 22

<210> 15

<211> 20

<212> DNA

<213> artificial sequence

<220>

<223> Forward primer/vorwärts Primer

<400> 15
tgaatatggg gctgaggaag 20

<210> 16

<211> 19

<212> DNA

<213> artificial sequence

<220>

<223> Backward primer/rückwärts Primer

<400> 16
ttctccctct gcctgtgtc 19

<210> 17

<211> 20

<212> DNA

<213> artificial sequence

<220>

<223> Forward primer/vorwärts Primer

<400> 17
ggtttagcac tgccttcagc 20

<210> 18

<211> 21

<212> DNA

<213> artificial sequence

<220>

<223> Backward primer/rückwärts Primer

<400> 18

cattaagcat ctggcatgtg g 21

<210> 19

<211> 20

<212> DNA

<213> artificial sequence

<220>

<223> Forward primer/vorwärts Primer

<400> 19
gaaaactcag attagcctgg 20

<210> 20

<211> 21

<212> DNA

<213> artificial sequence

<220>

<223> Backward primer/rückwärts Primer

<400> 20
atcttgagag caaaggttgt g 21

<210> 21

<211> 20

<212> DNA

<213> artificial sequence

<220>

<223> Forward primer/vorwärts Primer

<400> 21
tggttagggc atgattccag 20

<210> 22

<211> 22

<212> DNA

<213> artificial sequence

<220>

<223> Backward primer/rückwärts Primer

<400> 22
catgtataaa gagtaatgcc ag 22

<210> 23

<211> 19

<212> DNA

<213> artificial sequence

<220>

<223> Forward primer/vorwärts Primer

<400> 23
cgggagaggg tttgactac 19

<210> 24

<211> 21

<212> DNA

<213> artificial sequence

<220>

<223> Backward primer/rückwärts Primer

<400> 24
ctccgtattg ctcatctttc c 21

<210> 25

<211> 19

<212> DNA

<213> artificial sequence

<220>

<223> Forward primer/vorwärts Primer

<400> 25
agcttccctt ctctgagac 19

<210> 26

<211> 21

<212> DNA

<213> artificial sequence

<220>

<223> Backward primer/rückwärts Primer

<400> 26
gagaatagag tttgtgctca g 21

<210> 27

<211> 20

<212> DNA

<213> artificial sequence

<220>

<223> Forward primer/vorwärts Primer

<400> 27
gctaggaaaa atccgcaatg 20

<210> 28

<211> 19

<212> DNA

<213> artificial sequence

<220>

<223> Backward primer/rückwärts Primer

<400> 28
gaccccctag gacaccaac 19

<210> 29

<211> 20

<212> DNA

<213> artificial sequence

<220>

<223> Forward primer/vorwärts Primer

<400> 29
gagaattggg catgggcaga 20

<210> 30

<211> 21

<212> DNA

<213> artificial sequence

<220>

<223> Backward primer/rückwärts Primer

<400> 30
tgactttatc actggtcttt g 21

<210> 31

<211> 20

<212> DNA

<213> artificial sequence

<220>

<223> Forward primer/vorwärts Primer

<400> 31
ttatgcagcc catgacaatc 20

<210> 32

<211> 20

<212> DNA

<213> artificial sequence

<220>

<223> Backward primer/rückwärts Primer

<400> 32
caagggaact caaaggcttg 20

<210> 33

<211> 19

<212> DNA

<213> artificial sequence

<220>

<223> Forward primer/vorwärts Primer

<400> 33
tggaccaggt cagtttgtg 19

<210> 34

<211> 18

<212> DNA

<213> artificial sequence

<220>

<223> Backward primer/rückwärts Primer

<400> 34
tctgcctgtg tctctgcc 18

<210> 35

<211> 20

<212> DNA

<213> artificial sequence

<220>

<223> Forward primer/vorwärts Primer

<400> 35
atggcatgaa gaggataccg 20

<210> 36

<211> 20

<212> DNA

<213> artificial sequence

<220>

<223> Backward primer/rückwärts Primer

<400> 36
aggacaggtg acggctctac 20

<210> 37

<211> 20

<212> DNA

<213> artificial sequence

<220>

<223> Forward primer/vorwärts Primer

<400> 37
gctagtactc gattgtggtc 20

<210> 38

<211> 20

<212> DNA

<213> artificial sequence

<220>

<223> Backward primer/rückwärts Primer

<400> 38
tcatgggttg tgagatccag 20

<210> 39

<211> 20

<212> DNA

<213> artificial sequence

<220>

<223> Forward primer/vorwärts Primer

<400> 39
ttaatttgct cgtcttcctg 20

<210> 40

<211> 20

<212> DNA

<213> artificial sequence

<220>

<223> Backward primer/rückwärts Primer

<400> 40
tgtaagctcc acggatcacc 20

<210> 41

<211> 20

<212> DNA

<213> artificial sequence

<220>

<223> Forward primer/vorwärts Primer

<400> 41

ctctcttggt ctccctctgc 20

<210> 42

<211> 20

<212> DNA

<213> artificial sequence

<220>

<223> Backward primer/rückwärts Primer

<400> 42
gggagtaggg gtggagtagg 20

<210> 43

<211> 20

<212> DNA

<213> artificial sequence

<220>

<223> Forward primer/vorwärts Primer

<400> 43
ggtgtttcct ttccttttct 20

<210> 44

<211> 20

<212> DNA

<213> artificial sequence

<220>

<223> Backward primer/rückwärts Primer

<400> 44
ggtgttctct ccctttctct 20

<210> 45

<211> 20

<212> DNA

<213> artificial sequence

<220>

<223> Forward primer/vorwärts Primer

<400> 45
ctctatgaaa ggtgattgcc 20

<210> 46

<211> 21

<212> DNA

<213> artificial sequence

<220>

<223> Backward primer/rückwärts Primer

<400> 46
cagccataca aatgagaatt g 21

<210> 47

<211> 21

<212> DNA

<213> artificial sequence

<220>

<223> Forward primer/vorwärts Primer

<400> 47
ctacagtgaa tcagcacaga c 21

<210> 48

<211> 21

<212> DNA

<213> artificial sequence

<220>

<223> Backward primer/rückwärts Primer

<400> 48
cagccttgac tgtttctttg g 21

<210> 49

<211> 21

<212> DNA

<213> artificial sequence

<220>

<223> Forward primer/vorwärts Primer

<400> 49
tgattgactc tactttacac a 21

<210> 50

<211> 21

<212> DNA

<213> artificial sequence

<220>

<223> Backward primer/rückwärts Primer

<400> 50
tatattaggc ggttttcttc t 21

<210> 51

<211> 18

<212> DNA

<213> artificial sequence

<220>

<223> Forward primer/vorwärts Primer

<400> 51
acccaggtgg cctgattg 18

<210> 52

<211> 20

<212> DNA

<213> artificial sequence

<220>

<223> Backward primer/rückwärts Primer

<400> 52
gcacgcacgt tctctctttc 20

<210> 53

<211> 20

<212> DNA

<213> artificial sequence

<220>

<223> Forward primer/vorwärts Primer

<400> 53
gaatgccctt caccttgaaa 20

<210> 54

<211> 20

<212> DNA

<213> artificial sequence

<220>

<223> Backward primer/rückwärts Primer

<400> 54
ggaaaaggag agatgatgcc 20

<210> 55

<211> 21

<212> DNA

<213> artificial sequence

<220>

<223> Forward primer/vorwärts Primer

<400> 55
tcttccttgg aaagggaact c 21

<210> 56

<211> 20

<212> DNA

<213> artificial sequence

<220>

<223> Backward primer/rückwärts Primer

<400> 56
tgccctaaca cttggaatgg 20

<210> 57

<211> 23

<212> DNA

<213> artificial sequence

<220>

<223> Forward primer/vorwärts Primer

<400> 57
tggttaaaac attaaactta tag 23

<210> 58

<211> 21

<212> DNA

<213> artificial sequence

<220>

<223> Backward primer/rückwärts Primer

<400> 58
tagtatatag agatgcaatg g 21

<210> 59

<211> 20

<212> DNA

<213> artificial sequence

<220>

<223> Forward primer/vorwärts Primer

<400> 59
cattgggtgc tttcctgttc 20

<210> 60

<211> 19

<212> DNA

<213> artificial sequence

<220>

<223> Backward primer/rückwärts Primer

<400> 60
tggagctgca ggtatagcc 19

<210> 61

<211> 17

<212> DNA

<213> artificial sequence

<220>

<223> Forward primer/vorwärts Primer

<400> 61
ccaggcattc gagggtg 17

<210> 62

<211> 20

<212> DNA

<213> artificial sequence

<220>

<223> Backward primer/rückwärts Primer

<400> 62
cagaacttga ggaaccatag 20

<210> 63

<211> 21

<212> DNA

<213> artificial sequence

<220>

<223> Forward primer/vorwärts Primer

<400> 63
ccatgaactt tgtggaactg c 21

<210> 64

<211> 20

<212> DNA

<213> artificial sequence

<220>

<223> Backward primer/rückwärts Primer

<400> 64

aaagggttgc tgtggagatg 20

<210> 65

<211> 20

<212> DNA

<213> artificial sequence

<220>

<223> Forward primer/vorwärts Primer

<400> 65
aggcaggttc atctgtgtcc 20

<210> 66

<211> 20

<212> DNA

<213> artificial sequence

<220>

<223> Backward primer/rückwärts Primer

<400> 66
tcccagaccc agctacattc 20

<210> 67

<211> 21

<212> DNA

<213> artificial sequence

<220>

<223> Forward primer/vorwärts Primer

<400> 67
cagccaactg tattctcctt g 21

<210> 68

<211> 20

<212> DNA

<213> artificial sequence

<220>

<223> Backward primer/rückwärts Primer

<400> 68
atcttgagcc ctgcattagg 20

<210> 69

<211> 20

<212> DNA

<213> artificial sequence

<220>

<223> Forward primer/vorwärts Primer

<400> 69
gaagcgagga gagacagtcc 20

<210> 70

<211> 20

<212> DNA

<213> artificial sequence

<220>

<223> Backward primer/rückwärts Primer

<400> 70
aaggaagcct cctgacaacc 20

<210> 71

<211> 20

<212> DNA

<213> artificial sequence

<220>

<223> Forward primer/vorwärts Primer

<400> 71
cagggtcagg ctctatgctc 20

<210> 72

<211> 20

<212> DNA

<213> artificial sequence

<220>

<223> Backward primer/rückwärts Primer

<400> 72
tcctatcatc cggctttgac 20

<210> 73

<211> 20

<212> DNA

<213> artificial sequence

<220>

<223> Forward primer/vorwärts Primer

<400> 73
tctggattgt ggtcacaacc 20

<210> 74

<211> 22

<212> DNA

<213> artificial sequence

<220>

<223> Backward primer/rückwärts Primer

<400> 74
actggacact tcttttcaga cg 22

<210> 75

<211> 19

<212> DNA

<213> artificial sequence

<220>

<223> Forward primer/vorwärts Primer

<400> 75
aaagtggctg gttcggaag 19

<210> 76

<211> 20

<212> DNA

<213> artificial sequence

<220>

<223> Backward primer/rückwärts Primer

<400> 76
agcagcagca tacattcctc 20

<210> 77

<211> 21

<212> DNA

<213> artificial sequence

<220>

<223> Forward primer/vorwärts Primer

<400> 77
aagtagatcc tattatcgtg g 21

<210> 78

<211> 22

<212> DNA

<213> artificial sequence

<220>

<223> Backward primer/rückwärts Primer

<400> 78
agtttcagtg tctgttaaat ag 22

<210> 79

<211> 18

<212> DNA

<213> artificial sequence

<220>

<223> Forward primer/vorwärts Primer

<400> 79
ccggatttct gaggaggc 18

<210> 80

<211> 19

<212> DNA

<213> artificial sequence

<220>

<223> Backward primer/rückwärts Primer

<400> 80
catgctcttc accagaacc 19

<210> 81

<211> 22

<212> DNA

<213> artificial sequence

<220>

<223> Forward primer/vorwärts Primer

<400> 81
tcagcatgga ttttgtaaaa tc 22

<210> 82

<211> 18

<212> DNA

<213> artificial sequence

<220>

<223> Backward primer/rückwärts Primer

<400> 82
ggactgcgtg gacatctg 18

<210> 83

<211> 20

<212> DNA

<213> artificial sequence

<220>

<223> Forward primer/vorwärts Primer

<400> 83
acacggttct cgctgatgtg 20

<210> 84

<211> 20

<212> DNA

<213> artificial sequence

<220>

<223> Backward primer/rückwärts Primer

<400> 84
tgaaggggat tgaacagagg 20

<210> 85

<211> 21

<212> DNA

<213> artificial sequence

<220>

<223> Forward primer/vorwärts Primer

<400> 85
ttcattccga ggttctaact g 21

<210> 86

<211> 21

<212> DNA

<213> artificial sequence

<220>

<223> Backward primer/rückwärts Primer

<400> 86
caccatctcg tagcctttat c 21

<210> 87

<211> 22

<212> DNA

<213> artificial sequence

<220>

<223> Forward primer/vorwärts Primer

<400> 87

gcaagcttta aaataccttt cc 22

<210> 88

<211> 20

<212> DNA

<213> artificial sequence

<220>

<223> Backward primer/rückwärts Primer

<400> 88
gcctgaactg attgatgacc 20

<210> 89

<211> 22

<212> DNA

<213> artificial sequence

<220>

<223> Forward primer/vorwärts Primer

<400> 89
gggggaatta acaaagcctg ag 22

<210> 90

<211> 21

<212> DNA

<213> artificial sequence

<220>

<223> Backward primer/rückwärts Primer

<400> 90
tgcaaattct gagccccact g 21

<210> 91

<211> 22

<212> DNA

<213> artificial sequence

<220>

<223> Forward primer/vorwärts Primer

<400> 91
ctgttgagga acagagagaa gc 22

<210> 92

<211> 22

<212> DNA

<213> artificial sequence

<220>

<223> Backward primer/rückwärts Primer

<400> 92
tgtgttagaa tgttgcttcg ac 22

<210> 93

<211> 21

<212> DNA

<213> artificial sequence

<220>

<223> Forward primer/vorwärts Primer

<400> 93
tgagggctgc cactgtaaat a 21

<210> 94

<211> 17

<212> DNA

<213> artificial sequence

<220>

<223> Backward primer/rückwärts Primer

<400> 94
ggccccctca ccactcc 17

<210> 95

<211> 20

<212> DNA

<213> artificial sequence

<220>

<223> Forward primer/vorwärts Primer

<400> 95
atggccacat caccctagtc 20

<210> 96

<211> 22

<212> DNA

<213> artificial sequence

<220>

<223> Backward primer/rückwärts Primer

<400> 96
ctctctctgc atctctcatg aa 22

<210> 97

<211> 22

<212> DNA

<213> artificial sequence

<220>

<223> Forward primer/vorwärts Primer

<400> 97
taccactgtc atttttccat gc 22

<210> 98

<211> 19

<212> DNA

<213> artificial sequence

<220>

<223> Backward primer/rückwärts Primer

<400> 98
aagaggatac cggtggcag 19

<210> 99

<211> 21

<212> DNA

<213> artificial sequence

<220>

<223> Forward primer/vorwärts Primer

<400> 99
taaaggtcca ttgatagagg a 21

<210> 100

<211> 21

<212> DNA

<213> artificial sequence

<220>

<223> Backward primer/rückwärts Primer

<400> 100
tgatcccagg agttcattct t 21

<210> 101

<211> 23

<212> DNA

<213> artificial sequence

<220>

<223> Forward primer/vorwärts Primer

<400> 101
gactgagttc tttcagcaca gtg 23

<210> 102

<211> 21

<212> DNA

<213> artificial sequence

<220>

<223> Backward primer/rückwärts Primer

<400> 102
ggcagcctta ttattcatgg a 21

<210> 103

<211> 21

<212> DNA

<213> artificial sequence

<220>

<223> Forward primer/vorwärts Primer

<400> 103
cagttcatcc ttccccctct c 21

<210> 104

<211> 21

<212> DNA

<213> artificial sequence

<220>

<223> Backward primer/rückwärts Primer

<400> 104
gtgctagtct ggctgtgctc a 21

<210> 105

<211> 19

<212> DNA

<213> artificial sequence

<220>

<223> Forward primer/vorwärts Primer

<400> 105
tttggggcag ggagagtgg 19

<210> 106

<211> 20

<212> DNA

<213> artificial sequence

<220>

<223> Backward primer/rückwärts Primer

<400> 106
cccctgcagc aaatgtggtt 20

<210> 107

<211> 19

<212> DNA

<213> artificial sequence

<220>

<223> Forward primer/vorwärts Primer

<400> 107
agcaaggtcc acaagagcc 19

<210> 108

<211> 19

<212> DNA

<213> artificial sequence

<220>

<223> Backward primer/rückwärts Primer

<400> 108
gtagttccag tcacatcct 19

<210> 109

<211> 20

<212> DNA

<213> artificial sequence

<220>

<223> Forward primer/vorwärts Primer

<400> 109
caaagttcaa acaggaatgg 20

<210> 110

<211> 19

<212> DNA

<213> artificial sequence

<220>

<223> Backward primer/rückwärts Primer

<400> 110

gttctgtgct gagcattgg 19

<210> 111

<211> 24

<212> DNA

<213> artificial sequence

<220>

<223> Forward primer/vorwärts Primer

<400> 111
caaatgcttt agatcagaca tacg 24

<210> 112

<211> 21

<212> DNA

<213> artificial sequence

<220>

<223> Backward primer/rückwärts Primer

<400> 112
ctgaaaaatc tgtagcgatg c 21

<210> 113

<211> 20

<212> DNA

<213> artificial sequence

<220>

<223> Forward primer/vorwärts Primer

<400> 113
ccagattaac caggatgagg 20

<210> 114

<211> 20

<212> DNA

<213> artificial sequence

<220>

<223> Backward primer/rückwärts Primer

<400> 114
aatggcaagg atgctactcc 20

<210> 115

<211> 20

<212> DNA

<213> artificial sequence

<220>

<223> Forward primer/vorwärts Primer

<400> 115
gcccacagat aaggggtttt 20

<210> 116

<211> 20

<212> DNA

<213> artificial sequence

<220>

<223> Backward primer/rückwärts Primer

<400> 116
ggaagggaca gaagttttgc 20

<210> 117

<211> 20

<212> DNA

<213> artificial sequence

<220>

<223> Forward primer/vorwärts Primer

<400> 117
ctgctctttg taacccatgc 20

<210> 118

<211> 19

<212> DNA

<213> artificial sequence

<220>

<223> Backward primer/rückwärts Primer

<400> 118
aatgcctacc aggtgaagg 19

<210> 119

<211> 20

<212> DNA

<213> artificial sequence

<220>

<223> Forward primer/vorwärts Primer

<400> 119
accccctga tttgctttac 20

<210> 120

<211> 18

<212> DNA

<213> artificial sequence

<220>

<223> Backward primer/rückwärts Primer

<400> 120
agtcccggaa tcgagtcc 18

<210> 121

<211> 19

<212> DNA

<213> artificial sequence

<220>

<223> Forward primer/vorwärts Primer

<400> 121
ctggcagatt acaggtagc 19

<210> 122

<211> 18

<212> DNA

<213> artificial sequence

<220>

<223> Backward primer/rückwärts Primer

<400> 122
gttctccaaa gcactcat 18

<210> 123

<211> 23

<212> DNA

<213> artificial sequence

<220>

<223> Forward primer/vorwärts Primer

<400> 123
gactgagttc tttcagcaca gtg 23

<210> 124

<211> 21

<212> DNA

<213> artificial sequence

<220>

<223> Backward primer/rückwärts Primer

<400> 124
ggcagcctta ttattcatgg a 21

<210> 125

<211> 20

<212> DNA

<213> artificial sequence

<220>

<223> Forward primer/vorwärts Primer

<400> 125
ctatgtgcac gctgagagag 20

<210> 126

<211> 20

<212> DNA

<213> artificial sequence

<220>

<223> Backward primer/rückwärts Primer

<400> 126
tacccataaa gttgggcttg 20

<210> 127

<211> 20

<212> DNA

<213> artificial sequence

<220>

<223> Forward primer/vorwärts Primer

<400> 127
cttgatggca ccactctaat 20

<210> 128

<211> 20

<212> DNA

<213> artificial sequence

<220>

<223> Backward primer/rückwärts Primer

<400> 128
gtccctcaaa acacaaacat 20

<210> 129

<211> 21

<212> DNA

<213> artificial sequence

<220>

<223> Forward primer/vorwärts Primer

<400> 129
gcatgcaaag gtgttaattg g 21

<210> 130

<211> 19

<212> DNA

<213> artificial sequence

<220>

<223> Backward primer/rückwärts Primer

<400> 130
acacaaggaa gctttgggg 19

<210> 131

<211> 19

<212> DNA

<213> artificial sequence

<220>

<223> Forward primer/vorwärts Primer

<400> 131
atttgaagtc cgtggctcc 19

<210> 132

<211> 20

<212> DNA

<213> artificial sequence

<220>

<223> Backward primer/rückwärts Primer

<400> 132
gaaacaaaga ggcagcgatc 20

<210> 133

<211> 20

<212> DNA

<213> artificial sequence

<220>

<223> Forward primer/vorwärts Primer

<400> 133

aagcctattt ctccctctgc 20

<210> 134

<211> 20

<212> DNA

<213> artificial sequence

<220>

<223> Backward primer/rückwärts Primer

<400> 134
cattctattt gctcctgtgg 20

<210> 135

<211> 24

<212> DNA

<213> artificial sequence

<220>

<223> Forward primer/vorwärts Primer

<400> 135
ggttattgta aagtctgagt gttg 24

<210> 136

<211> 25

<212> DNA

<213> artificial sequence

<220>

<223> Backward primer/rückwärts Primer

<400> 136
aatagactat gtaactgtct ctggc 25

<210> 137

<211> 24

<212> DNA

<213> artificial sequence

<220>

<223> Forward primer/vorwärts Primer

<400> 137
aaaaagtgta gagctttctt caaa 24

<210> 138

<211> 23

<212> DNA

<213> artificial sequence

<220>

<223> Backward primer/rückwärts Primer

<400> 138
attgagaccc aagactgtta gtg 23

<210> 139

<211> 22

<212> DNA

<213> artificial sequence

<220>

<223> Forward primer/vorwärts Primer

<400> 139
taaattcaat aagccatgga gg 22

<210> 140

<211> 22

<212> DNA

<213> artificial sequence

<220>

<223> Backward primer/rückwärts Primer

<400> 140
ggtaacaacc agagtgtgtg tg 22

<210> 141

<211> 4

<212> DNA

<213> Struktureinheit des Mikrosatelliten/repeating units of micro satellite

<220>

<221> repeat_unit

<222> (1)..(4)

<223>

<400> 141
ttta 4

<210> 142

<211> 6

<212> DNA

<213> Struktureinheit des Mikrosatelliten/repeating units of micro satellite

<220>

<221> repeat_unit

<222> (1)..(3)

<223>

<220>

<221> repeat_unit

<222> (4)..(6)

<223>

<400> 142
accatc 6

<210> 143

<211> 8

<212> DNA

<213> Struktureinheit des Mikrosatelliten/repeating units of micro satellite

<220>

<221> repeat_unit

<222> (1)..(4)

<223>

<220>

<221> repeat_unit

<222> (5)..(8)

<223>

<400> 143
atttgttt 8

<210> 144

<211> 2

<212> DNA

<213> Struktureinheit des Mikrosatelliten/repeating units of micro satellite

<220>

<221> repeat_unit

<222> (1)..(2)

<223>

<400> 144
ca 2

<210> 145

<211> 2

<212> DNA

<213> Struktureinheit des Mikrosatelliten/repeating units of micro satellite

<220>

<221> repeat_unit

<222> (1)..(2)

<223>

<400> 145
ga 2

<210> 146

<211> 4

<212> DNA

<213> Struktureinheit des Mikrosatelliten/repeating units of micro satellite

<220>

<221> repeat_unit

<222> (1)..(4)

<223>

<400> 146
cttt 4

<210> 147

<211> 2

<212> DNA

<213> Struktureinheit des Mikrosatelliten/repeating units of micro satellite

<220>

<221> repeat_unit

<222> (1)..(2)

<223>

<400> 147
ga 2

<210> 148

<211> 4

<212> DNA

<213> Struktureinheit des Mikrosatelliten/repeating units of micro satellite

<220>

<221> repeat_unit

<222> (1)..(2)

<223>

<220>

<221> repeat_unit

<222> (3)..(4)

<223>

<400> 148
acag 4

<210> 149

<211> 6

<212> DNA

<213> Struktureinheit des Mikrosatelliten/repeating units of micro satellite

<220>

<221> repeat_unit

<222> (1)..(2)

<223>

<220>

<221> repeat_unit

<222> (5)..(6)

<223>

<400> 149
ctttct 6

<210> 150

<211> 5

<212> DNA

<213> Struktureinheit des Mikrosatelliten/repeating units of micro satellite

<220>

<221> repeat_unit

<222> (1)..(5)

<223>

<400> 150
aaaat 5

<210> 151

<211> 6

<212> DNA

<213> Struktureinheit des Mikrosatelliten/repeating units of micro satellite

<220>
<221> repeat_unit
<222> (1)..(2)
<223>

<220>
<221> repeat_unit
<222> (3)..(4)
<223>

<220>
<221> repeat_unit
<222> (5)..(6)
<223>

<400> 151
ctgtct 6

<210> 152
<211> 5
<212> DNA
<213> Struktureinheit des Mikrosatelliten/repeating units of micro satellite

<220>
<221> repeat_unit
<222> (1)..(4)
<223>

<220>
<221> repeat_unit
<222> (5)..(5)
<223>

<400> 152
atttt 5

<210> 153

<211> 2

<212> DNA

<213> Struktureinheit des Mikrosatelliten/repeating units of micro satellite

<220>

<221> repeat_unit

<222> (1)..(2)

<223>

<400> 153
ac 2

<210> 154

<211> 4

<212> DNA

<213> Struktureinheit des Mikrosatelliten/repeating units of micro satellite

<220>

<221> repeat_unit

<222> (1)..(4)

<223>

<400> 154
taga 4

<210> 155

<211> 2

<212> DNA

<213> Struktureinheit des Mikrosatelliten/repeating units of micro satellite

<220>

<221> repeat_unit

<222> (1)..(2)

<223>

<400> 155
ga 2

<210> 156

<211> 4

<212> DNA

<213> Struktureinheit des Mikrosatelliten/repeating units of micro satellite

<220>

<221> repeat_unit

<222> (1)..(2)

<223>

<220>

<221> repeat_unit

<222> (3)..(4)

<223>

<400> 156
gtat 4

<210> 157

<211> 2

<212> DNA

<213> Struktureinheit des Mikrosatelliten/repeating units of micro satellite

<220>

<221> repeat_unit

<222> (1)..(2)

<223>

<400> 157
ag 2

<210> 158

<211> 2

<212> DNA

<213> Struktureinheit des Mikrosatelliten/repeating units of micro satellite

<220>

<221> repeat_unit

<222> (1)..(2)

<223>

<400> 158
gt 2

<210> 159

<211> 2

<212> DNA

<213> Struktureinheit des Mikrosatelliten/repeating units of micro satellite

<220>

<221> repeat_unit

<222> (1)..(2)

<223>

<400> 159
ag 2

<210> 160

<211> 2

<212> DNA

<213> Struktureinheit des Mikrosatelliten/repeating units of micro satellite

<220>

<221> repeat_unit

<222> (1)..(2)

<223>

<400> 160
ca 2

<210> 161

<211> 2

<212> DNA

<213> Struktureinheit des Mikrosatelliten/repeating units of micro satellite

<220>

<221> repeat_unit

<222> (1)..(2)

<223>

<400> 161
ga 2

<210> 162

<211> 4

<212> DNA

<213> Struktureinheit des Mikrosatelliten/repeating units of micro satellite

<220>

<221> repeat_unit

<222> (1)..(4)

<223>

<400> 162
cttt 4

<210> 163

<211> 4

<212> DNA

<213> Struktureinheit des Mikrosatelliten/repeating units of micro satellite

<220>

<221> repeat_unit

<222> (1)..(4)

<223>

<400> 163
gaaa 4

<210> 164

<211> 2

<212> DNA

<213> Struktureinheit des Mikrosatelliten/repeating units of micro satellite

<220>

<221> repeat_unit

<222> (1)..(2)

<223>

<400> 164
gt 2

<210> 165

<211> 2

<212> DNA

<213> Struktureinheit des Mikrosatelliten/repeating units of micro satellite

<220>

<221> repeat_unit

<222> (1)..(2)

<223>

<400> 165
ca 2

<210> 166

<211> 2

<212> DNA

<213> Struktureinheit des Mikrosatelliten/repeating units of micro satellite

<220>

<221> repeat_unit

<222> (1)..(2)

<223>

<400> 166
ag 2

<210> 167

<211> 4

<212> DNA

<213> Struktureinheit des Mikrosatelliten/repeating units of micro satellite

<220>

<221> repeat_unit

<222> (1)..(4)

<223>

<400> 167
gaaa 4

<210> 168

<211> 2

<212> DNA

<213> Struktureinheit des Mikrosatelliten/repeating units of micro satellite

<220>

<221> repeat_unit

<222> (1)..(2)

<223>

<400> 168
ac 2

<210> 169

<211> 2

<212> DNA

<213> Struktureinheit des Mikrosatelliten/repeating units of micro satellite

<220>

<221> repeat_unit

<222> (1)..(2)

<223>

<400> 169
tc 2

<210> 170

<211> 2

<212> DNA

<213> Struktureinheit des Mikrosatelliten/repeating units of micro satellite

<220>

<221> repeat_unit

<222> (1)..(2)

<223>

<400> 170
ac 2

<210> 171

<211> 2

<212> DNA

<213> Struktureinheit des Mikrosatelliten/repeating units of micro satellite

<220>

<221> repeat_unit

<222> (1)..(2)

<223>

<400> 171
gt 2

<210> 172

<211> 2

<212> DNA

<213> Struktureinheit des Mikrosatelliten/repeating units of micro satellite

<220>

<221> repeat_unit

<222> (1)..(2)

<223>

<400> 172
ac 2

<210> 173

<211> 2

<212> DNA

<213> Struktureinheit des Mikrosatelliten/repeating units of micro satellite

<220>

<221> repeat_unit

<222> (1)..(2)

<223>

<400> 173
gt 2

<210> 174

<211> 13

<212> DNA

<213> Struktureinheit des Mikrosatelliten/repeating units of micro satellite

<220>

<221> repeat_unit

<222> (1)..(2)

<223>

<220>

<221> repeat_unit

<222> (3)..(4)

<223>

<220>

<221> repeat_unit

<222> (5)..(6)

<223>

<220>

<221> repeat_unit

<222> (7)..(8)

<223>

<220>

<221> repeat_unit

<222> (10)..(13)

<223>

<400> 174
tatgtacact aaa 13

<210> 175

<211> 3

<212> DNA

<213> Struktureinheit des Mikrosatelliten/repeating units of micro satellite

<220>

<221> repeat_unit

<222> (1)..(3)

<223>

<400> 175
aat 3

<210> 176

<211> 2

<212> DNA

<213> Struktureinheit des Mikrosatelliten/repeating units of micro satellite

<220>

<221> repeat_unit

<222> (1)..(2)

<223>

<400> 176
ct 2

<210> 177

<211> 2

<212> DNA

<213> Struktureinheit des Mikrosatelliten/repeating units of micro satellite

<220>

<221> repeat_unit

<222> (1)..(2)

<223>

<400> 177
tg 2

<210> 178

<211> 2

<212> DNA

<213> Struktureinheit des Mikrosatelliten/repeating units of micro satellite

<220>

<221> repeat_unit

<222> (1)..(2)

<223>

<400> 178
ct 2

<210> 179

<211> 4

<212> DNA

<213> Struktureinheit des Mikrosatelliten/repeating units of micro satellite

<220>

<221> repeat_unit

<222> (1)..(4)

<223>

<400> 179
taaa 4

<210> 180

<211> 6

<212> DNA

<213> Struktureinheit des Mikrosatelliten/repeating units of micro satellite

<220>

<221> repeat_unit

<222> (1)..(2)

<223>

<220>

<221> repeat_unit

<222> (5)..(6)

<223>

<400> 180
gtccgt 6

<210> 181

<211> 4

<212> DNA

<213> Struktureinheit des Mikrosatelliten/repeating units of micro satellite

<220>

<221> repeat_unit

<222> (1)..(4)

<223>

<400> 181
attt 4

<210> 182

<211> 8

<212> DNA

<213> Struktureinheit des Mikrosatelliten/repeating units of micro satellite

<220>

<221> repeat_unit

<222> (1)..(2)

<223>

<220>

<221> repeat_unit

<222> (3)..(4)

<223>

<220>

<221> repeat_unit

<222> (5)..(6)

<223>

<220>

<221> repeat_unit

<222> (7)..(8)

<223>

<400> 182
tccacgca 8

<210> 183

<211> 2

<212> DNA

<213> Struktureinheit des Mikrosatelliten/repeating units of micro satellite

<220>

<221> repeat_unit

<222> (1)..(2)

<223>

<400> 183
gt 2